# EUROPEAN PATENT SPECIFICATION

(11) **EP 3 285 572 B1**
(45) Date of publication and mention of the grant of the patent: **02.12.2020**
(21) Application number: 16721711.6
(22) Date of filing: 21.04.2016
(51) Int. Cl.: A61K 31/409, A61P 3/00, A61P 3/10, C12N 5/071, A01K 67/027

(54) **METHOD FOR PRODUCTION OF INSULIN-PRODUCING CELLS**
VERFAHREN ZUR HERSTELLUNG VON INSULINPRODUZIERENDEN ZELLEN
PROCÉDÉ DE PRODUCTION DE CELLULES PRODUISANT DE L'INSULINE

(30) Priority: 24.04.2015 EP 15164989
(43) Date of publication of application: 28.02.2018
(73) Proprietor: University of Copenhagen, 1165 Copenhagen K (DK)
(72) Inventor: MAMIDI, Anant, 2650 Hvidovre (DK); SEMB, Henrik, 23735 Bjärred (SE)
(74) Representative: Høiberg P/S
(86) International application number: PCT/EP2016/058915
(87) International publication number: WO 2016/170067

(56) References cited:
- WO-A1-2013/163739
- WO-A1-2013/188138
- WO-A1-2014/201167
- WO-A2-2007/130474
- D'AMOUR K A ET AL: "Production of pancreatic hormone-expressing endocrine cells from human embryonic stem cells", NATURE BIOTECHNOLOGY, NATURE PUBLISHING GROUP, US, vol. 24, no. 11, 1 January 2006 (2006-01-01), pages 1392-1401, XP002423119, ISSN: 1087-0156, DOI: 10.1038/NBT1259
- DIEP CAROLINE H ET AL: "Down-regulation of Yes Associated Protein 1 expression reduces cell proliferation and clonogenicity of pancreatic cancer cells.", PLOS ONE 2012, vol. 7, no. 3, E32783, 2012, pages 1-9, XP002742178, ISSN: 1932-6203
- S. KUMAR ET AL: "Recent Developments in [beta]-Cell Differentiation of Pluripotent Stem Cells Induced by Small and Large Molecules", INTERNATIONAL JOURNAL OF MOLECULAR SCIENCES, vol. 15, no. 12, 17 December 2014 (2014-12-17), pages 23418-23447, XP055167269, DOI: 10.3390/ijms151223418
- ZHANG ZHEN-WU ET AL: "miR-375 Inhibits Proliferation of Mouse Pancreatic Progenitor Cells by Targeting YAP1", CELLULAR PHYSIOLOGY AND BIOCHEMISTRY, vol. 32, no. 6, 2013, pages 1808-1817, XP002742179,
- CEBOLA INÊS ET AL: "TEAD and YAP regulate the enhancer network of human embryonic pancreatic progenitors.", NATURE CELL BIOLOGY MAY 2015, vol. 17, no. 5, May 2015 (2015-05), pages 615-626, XP002742180, ISSN: 1476-4679

## Description

### Technical field

The present disclosure relates to a method for differentiation of cells into insulin-producing cells, to a cell population enriched for insulin-producing β-cells and to the use of such a cell population for treatment of a metabolic disorder in an individual in need thereof. Also disclosed is a method for treating a metabolic disorder.

### Background

Cell therapy treatment of insulin dependent diabetes is facilitated by the production of unlimited numbers of pancreatic cells that can and will be able to function similarly to human islets. For example, the use of insulin-producing β-cells derived from human embryonic stem cells (hESCs) would offer a vast improvement over current cell therapy procedures that utilize cells from donor pancreases. Currently cell therapy treatments for diabetes mellitus, such as type 1 or type 2 diabetes, which utilize cells from donor pancreases, are limited by the scarcity of high quality islet cells needed for transplant. For example, cell therapy for a single type 1 diabetic patient requires a transplant of approximately 8 x 10⁸ pancreatic islet cells (Shapiro et al, 2000, N Engl J Med 343:230-238; Shapiro et al, 2001a, Best Pract Res Clin Endocrinol Metab 15:241- 264; Shapiro etal, 2001b, British Medical Journal 322:861). As such, at least two healthy donor organs are required to obtain sufficient islet cells for a successful transplant.

Embryonic stem (ES) cells thus represent a powerful model system for the investigation of mechanisms underlying pluripotent cell biology and differentiation within the early embryo, as well as providing opportunities for genetic manipulation of mammals and resultant commercial, medical and agricultural applications. Furthermore, appropriate proliferation and differentiation of ES cells can potentially be used to generate an unlimited source of cells suited to transplantation for treatment of diseases that result from cell damage or dysfunction. Other pluripotent cells and cell lines including early primitive ectoderm-like (EPL) cells, in vivo or in vitro derived ICM/epiblast, in vivo or in vitro derived primitive ectoderm, primordial germ cells (EG cells), teratocarcinoma cells (EC cells), and pluripotent cells derived by dedifferentiation or by nuclear transfer can also be used.

D'Amour et al. (2006) describe a five-step protocol for differentiation of ES cells into insulin producing cells. WO 2014/201167 discloses the generation of functional human beta cells from human pluripotent stem cells and hiPSCs. WO 2013/163739 describes the generation of NKX6-1+ cells from human ES cells and their further differentiation into insulin-producing cells. WO 2007/130474 discloses methods for differentiation of hESCs to beta-cells. WO 2013/188138 discloses method for slowing down or preventing cancer and tumor progression by inhibition of the Hippo/YAP signalling pathway.

Accordingly, there is a need for producing these pancreatic type cells derived from hES cells, as well as reliable methods for purifying such cells.

### Summary

Herein disclosed are methods for differentiation of cells into insulin-producing β-cells, a cell population enriched for insulin-producing β-cells obtainable by the methods described herein, as well as methods for treating a metabolic disorder.

### Description of Drawings

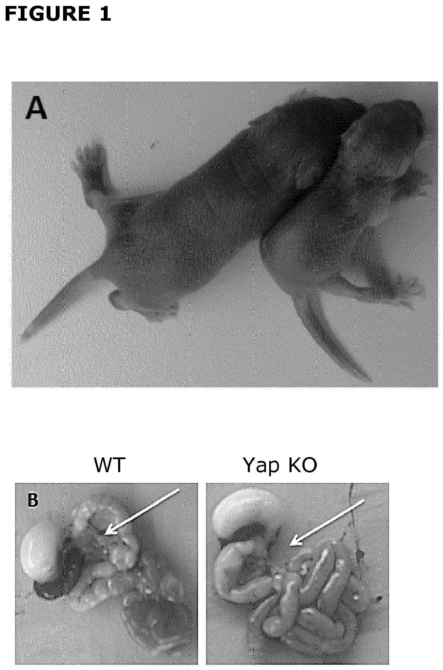

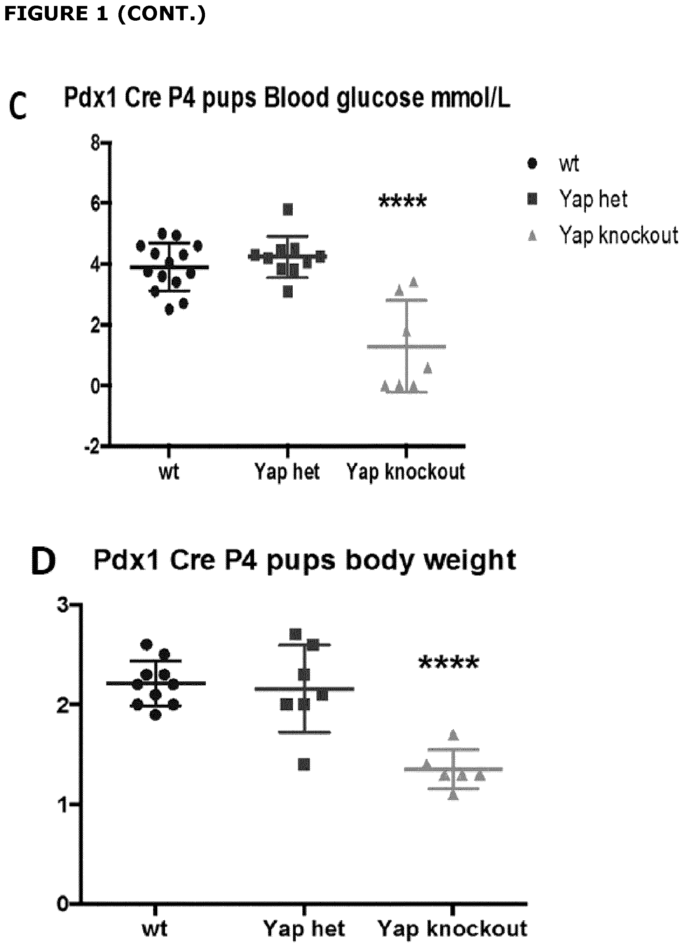

**Figure 1****: Pdx1 Cre Yap1 knockout mice.** (A) Picture of P4 pups from control and Yap1 knockout (Yap KO) mice. The Yap KO pups display smaller body size than control littermates. (B) Viscera from Control wild-types and Yap Knockout mice. The Yap KO display severe pancreatic hypoplasia with a pancreatic mass reduced by at least one third compared to control littermates. (C) Yap KO pups are severely hypoglycemic. Blood glucose levels were measured from post natal day P4 before the pups were sacrificed and the pancreata collected for histological examination. Yap het: Yap1^{fl/+}. Y-axis: blood glucose (mmol/L). (D) Yap KO pups have significantly reduced body weight at P4. Y-axis: body weight in grams.

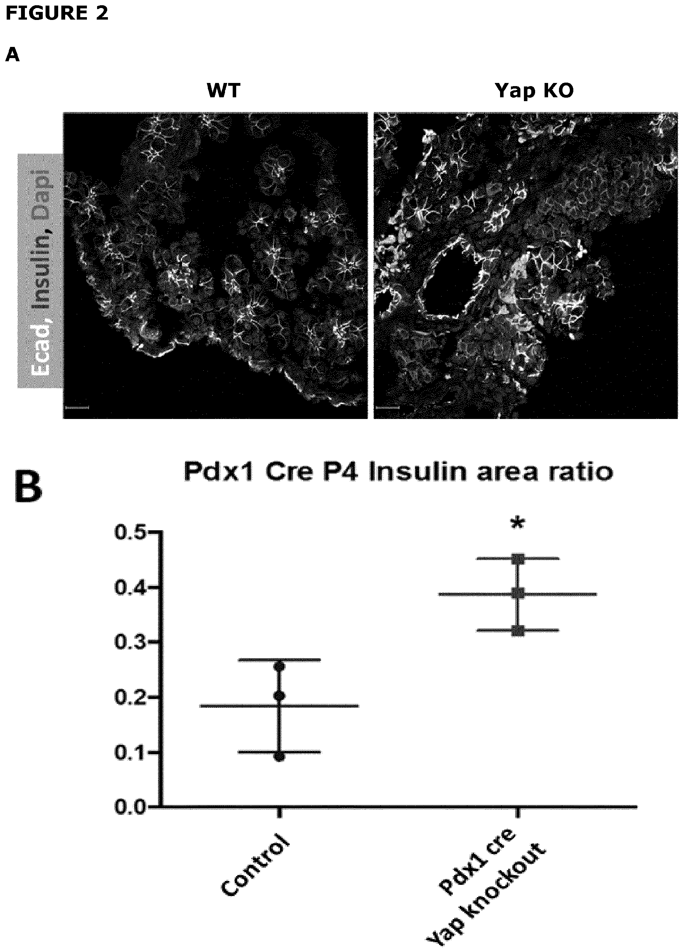

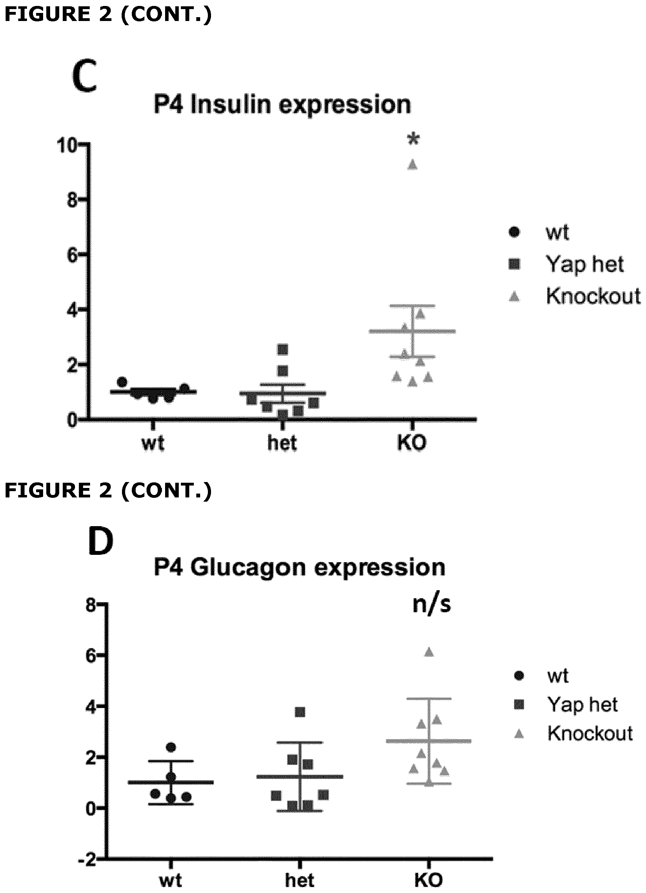

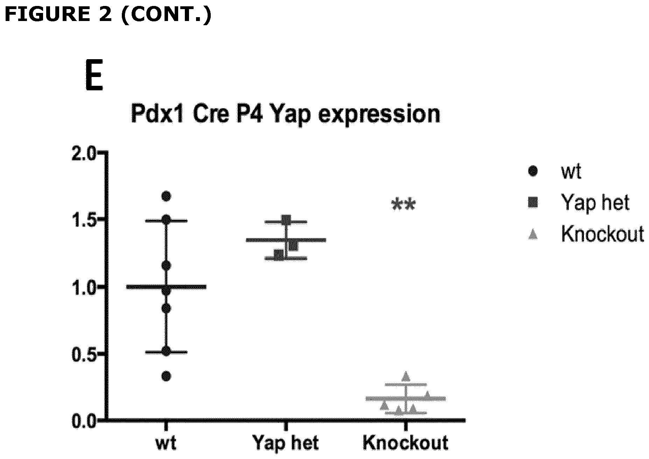

**Figure 2****: Pdx1 Cre Yap1 Knockout pups display elevated insulin expression.** (A) Confocal images of controls and Yap Knockout P4 pancreas stained with DAPI (light grey), Ecad (white) and Insulin (dark grey), show elevated insulin area in the Knockouts despite the hypoplastic pancreas phenotype. (B) P4 wild type and Yap knockout pancreas sections were immuno-stained with antibodies against Ecad, Insulin and DAPI in fig. 2A. The ratio of insulin expressing area to the total DAPI area of pancreas was calculated. In the Yap knockout insulin expression/endocrine differentiation was significantly increased (n=3). Y-axis: insulin area/DAPI area. (C) RT-qPCR analysis from P4 pancreata shows a significant increase in mRNA levels of Insulin in Yap Knockout pancreas. Y-axis: relative gene expression. (D) Glucagon mRNA expression is not significantly altered in Yap Knockouts compared to controls. (E) Yap1 mRNA levels are significantly down-regulated in the knockout pancreas compared to littermate controls.

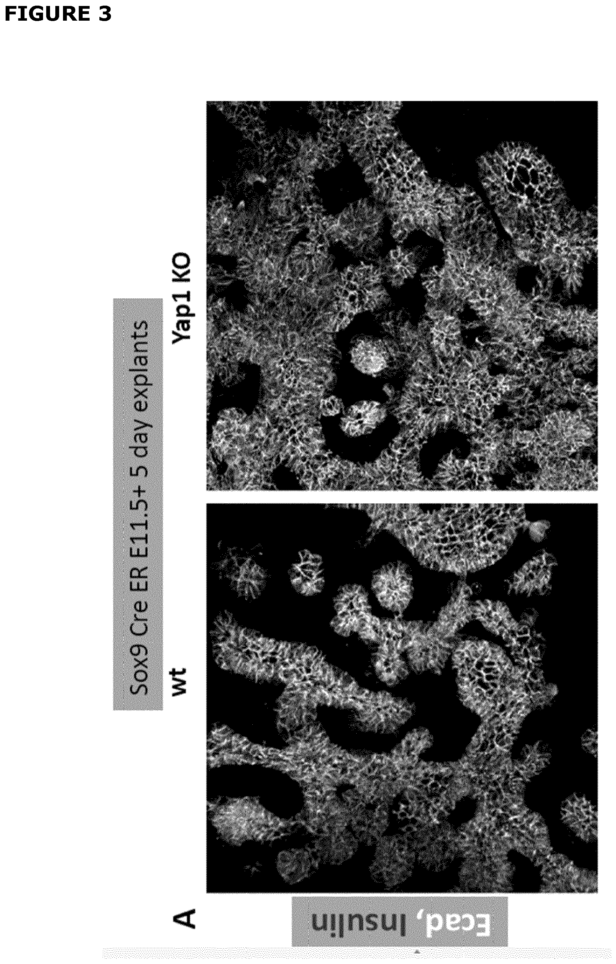

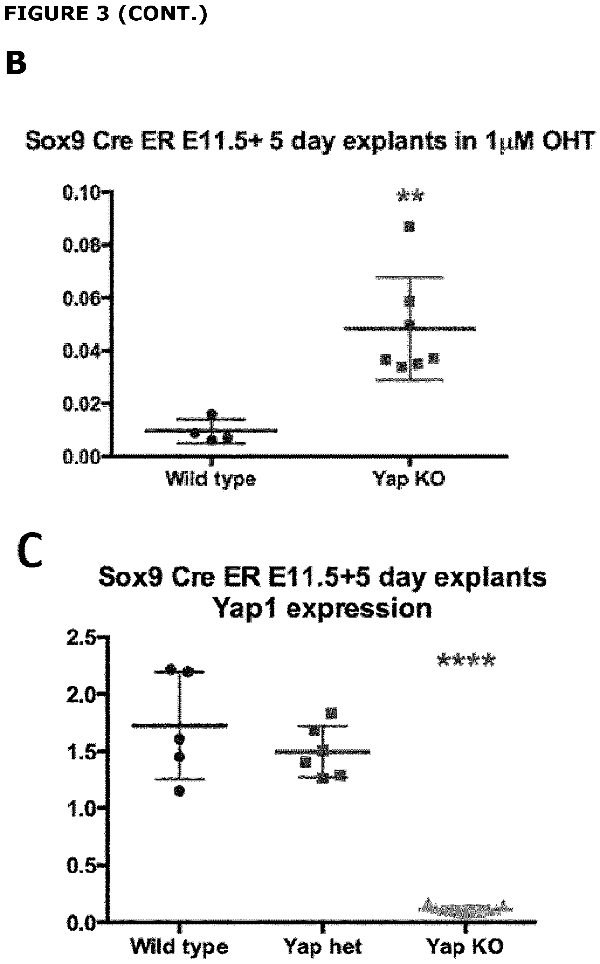

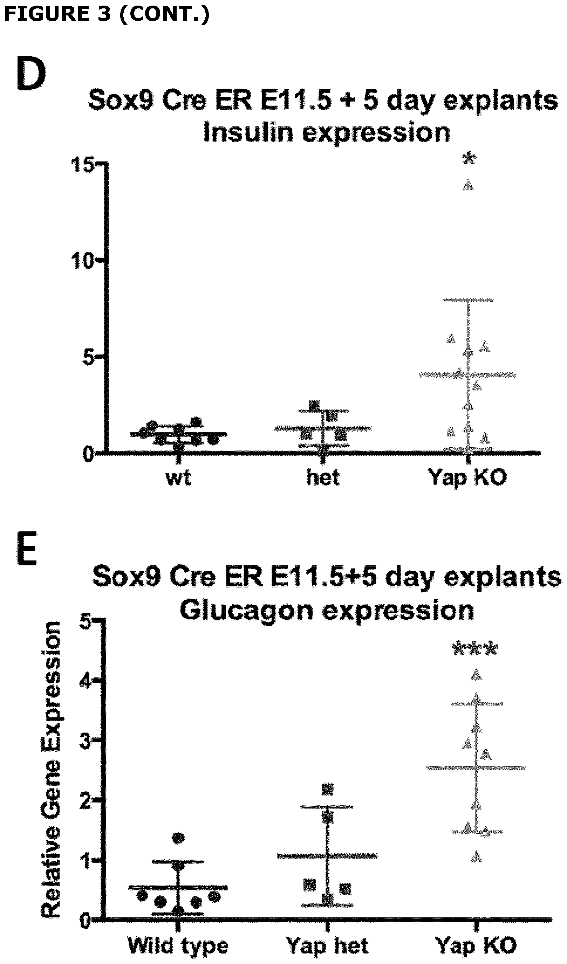

**Figure 3****: Loss of Yap1 in Sox9 expressing pancreatic progenitors leads to elevated insulin expression.** (A) Confocal z-stack images of controls (wtild type, wt) and Yap1 Knockout (Yap1 KO) Sox9 Cre ER E11.5 mouse pancreatic explants grown ex-vivo for 5 days in presence of 1uM OHT stained with E-cadherin and Insulin show elevated insulin expressing area in the Yap1 Knockouts. (B) Confocal Z-stack images from figure A of Sox9 Cre ER wild type and Yap1 knockout pancreas explants were quantified for ratio of insulin expressing volume to the total E-cadherin expressing volume. In the Yap1 knockout, insulin expression/endocrine differentiation was significantly increased (n=7). Y-axis: insulin area/Ecad area (µm²). (C) RT-qPCR analysis from Sox9 Cre ER E11.5 + 5 days explants shows a significant reduction in mRNA levels of Yap1 in Yap1 Knockout explants. Yap het = heterozygote Yap1^{fl/+}. (D) Insulin mRNA expression is significantly up-regulated in Yap1 knockouts compared to controls. (E) Glucagon mRNA levels are significantly up-regulated in the knockout explants compared to controls. (C-E): Y-axis: relative gene expression (fold change versus wild type).

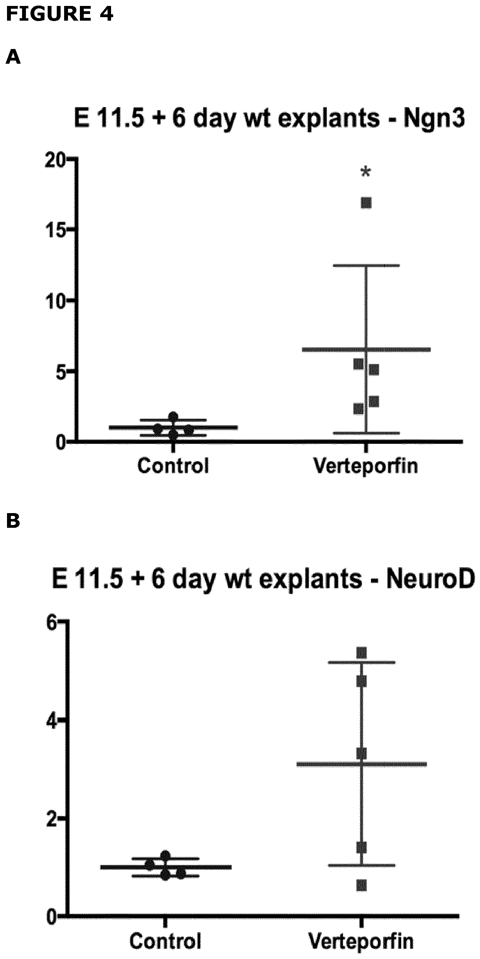

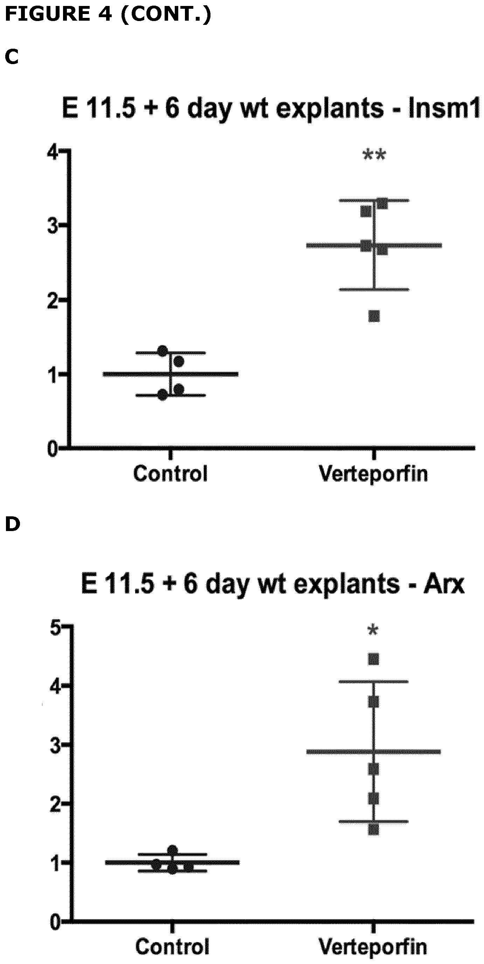

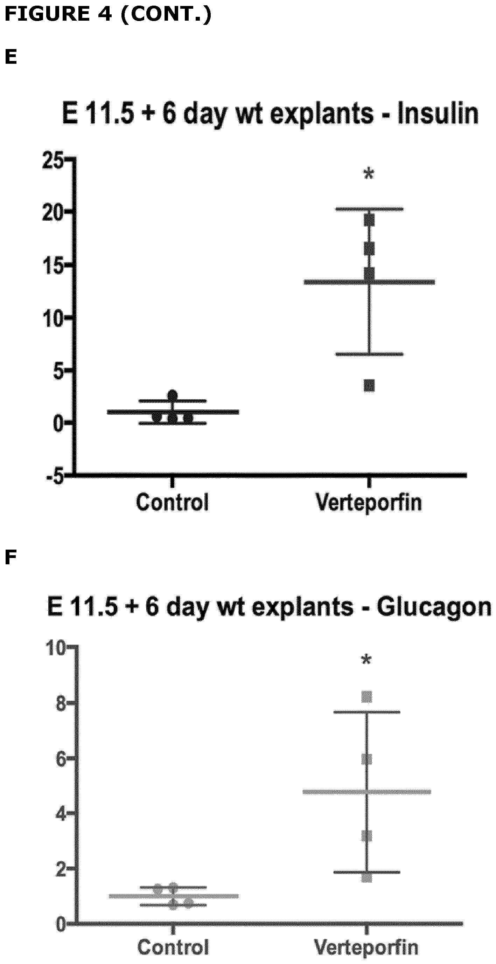

**Figure 4****: Inhibition of Yap1 using Verteporfin in wild type mouse pancreatic explants leads to enhanced endocrine differentiation.** Wild type C57bl6 mouse pancreatic explants at E11.5 embryonic stages were cultured ex-vivo for initial 24hrs in normal culture media supplemented with 1ug/ml Verteporfin for 72 hours and again in normal culture media without Verteporfin for another 48 hours. The explants were analyzed by RT-qPCR for endocrine gene expression after 6 days of culture. Inhibition of Yap1 using Verteporfin leads to significant up-regulation of endocrine progenitors Neurogenin (Ngn3) (A), NeuroD (B), Insm1 (C), Arx expression (D). This ultimately leads to enhanced endocrine differentiation as analysed for Insulin (E) and Glucagon (F) expression. Y axis: relative expression (fold change relative to Ecad expression).

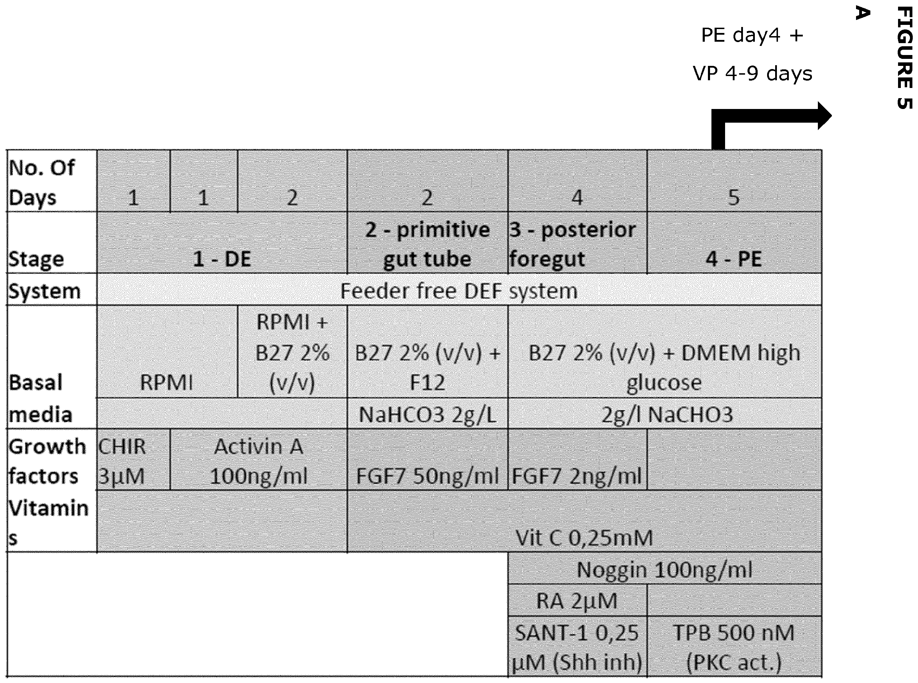

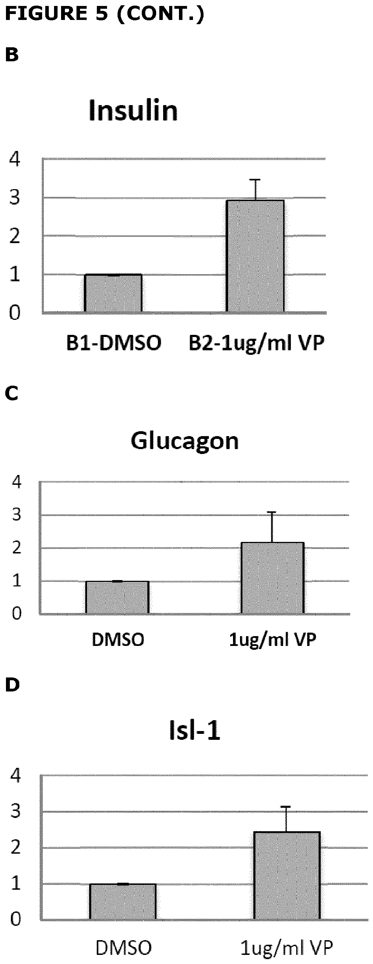

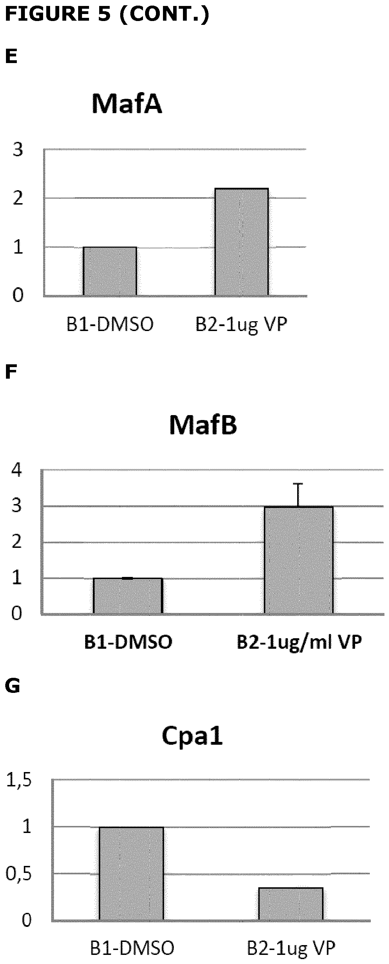

**Figure 5****: Inhibition of YAP1 in hES cell derived pancreatic progenitors leads to β-cell differentiation.** (A) Outline of the 15 day protocol (modified from Rezania et al, 2010) used to generate pancreatic progenitors from human ES cells. At the end of the protocol the majority of cells express the progenitor markers PDX1 and NKX6.1. The YAP1-TEAD inhibitor Verteporfin (VP) was added to the medium during PE day 4 and the cells were treated for either 4 or 5 days. (B, C, D, E, F, G) Real time PCR analysis of expression of endocrine and beta cell specific genes from the 4 day verteporfin-treated cells shows increased expression of Insulin, Glucagon, ISL-1, MAFA, MAFB but decreased expression of the exocrine gene CPA1. Y axis: relative mRNA expression as fold change.

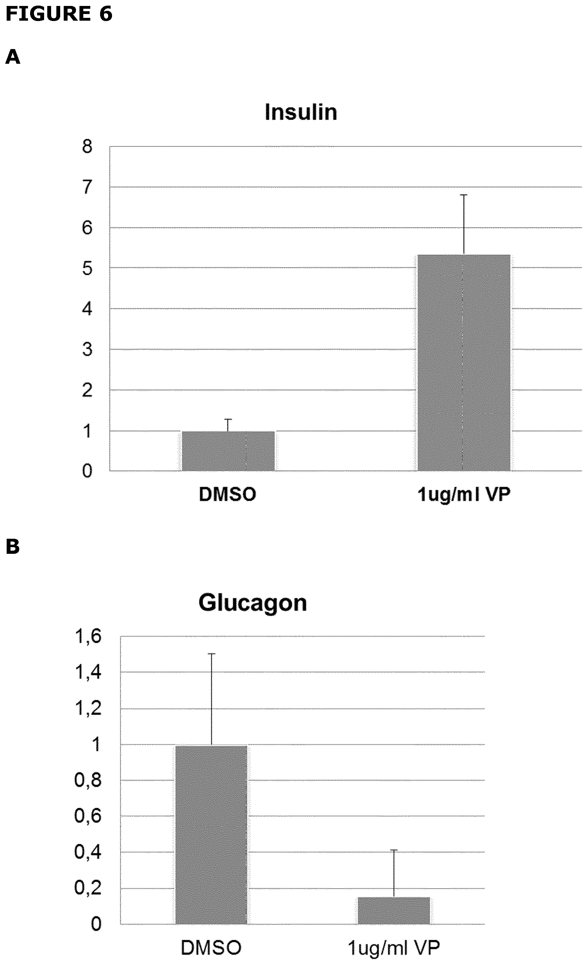

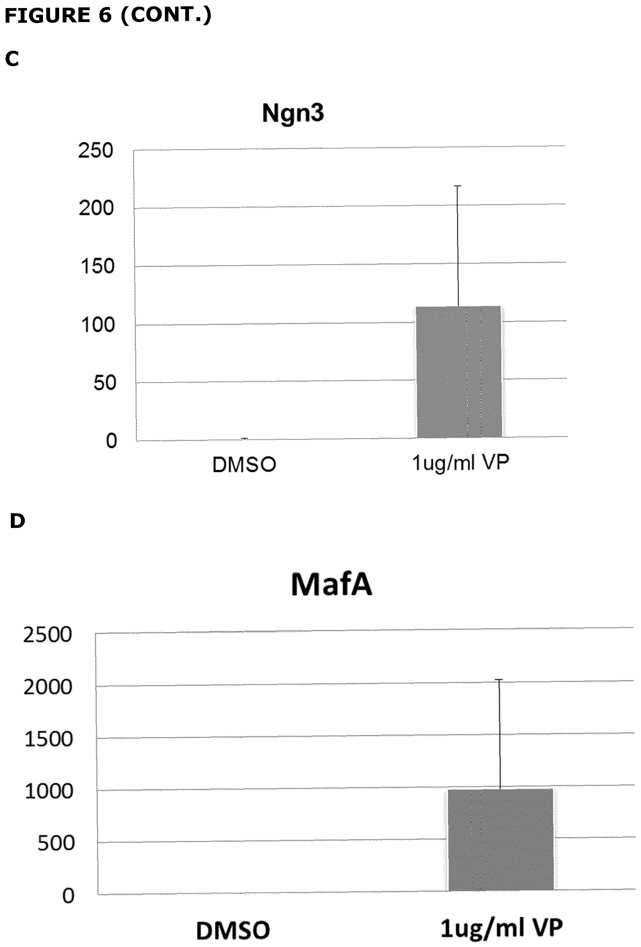

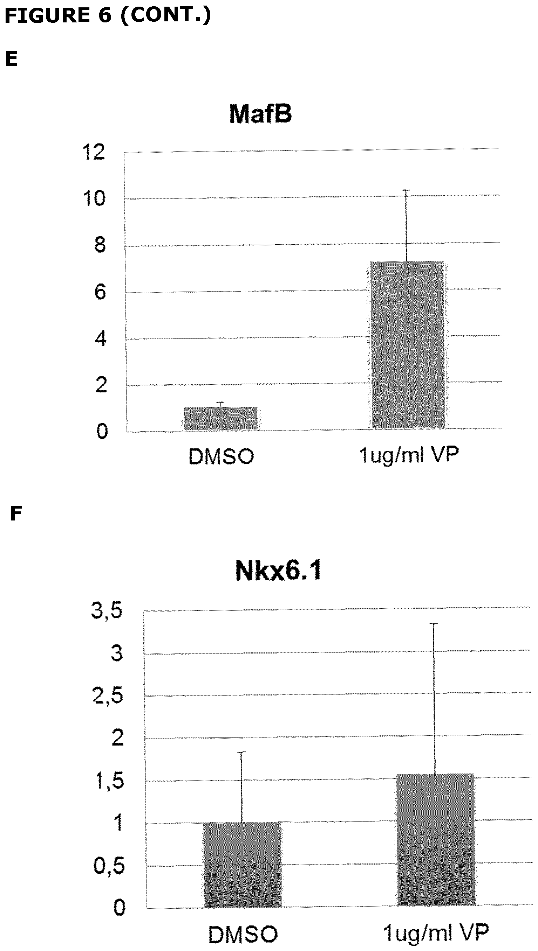

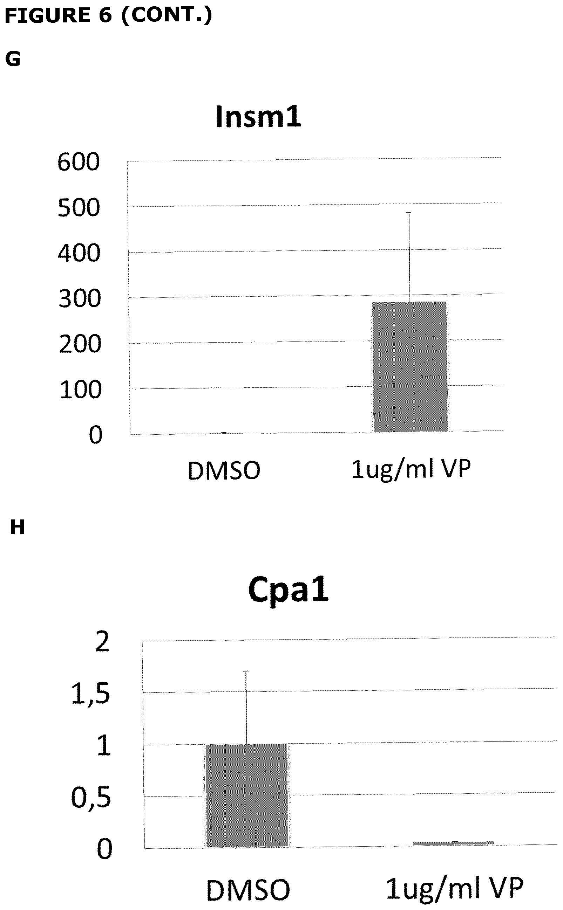

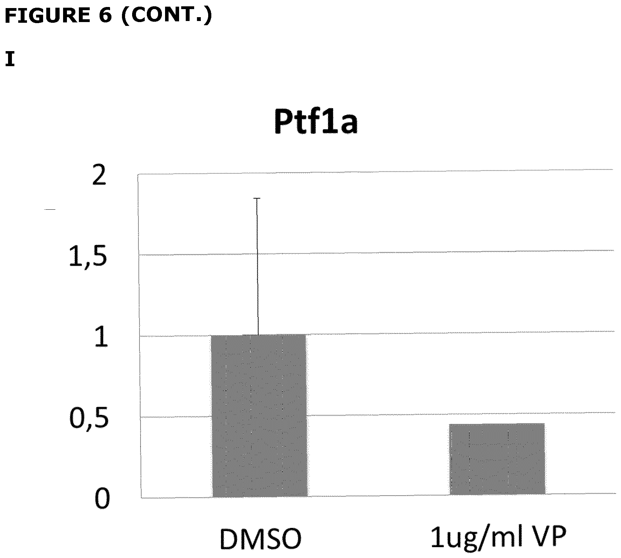

**Figure 6****: Treatment with verteporfin for 5 days leads to enhanced expression of islet specific genes.** Real time PCR analysis of pancreatic progenitors treated with verteporfin for 5 days shows up-regulation of endocrine progenitor gene Neurogenin3 (NGN3), beta- cell specific genes Insulin, MAFA, MAFB, INSM1, down-regulation of Glucagon and exocrine specific genes CPA1 and PTF1a. Expression of NKX6.1 is not altered, as it is expressed in both pancreatic progenitors and mature beta-cells. Y-axis: relative mRNA expression (fold-change versus DMSO control).

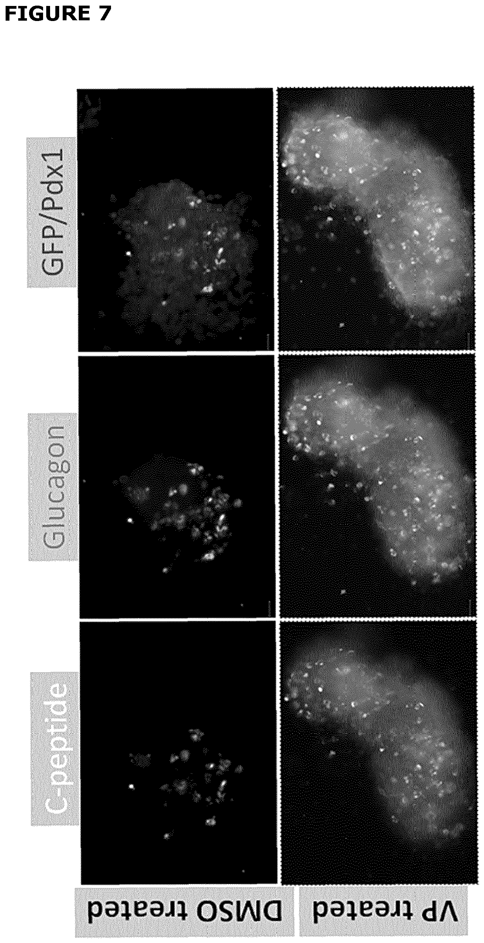

**Figure 7****: Treating hES cell derived pancreatic progenitors with verteporfin for 5 days leads to enhanced insulin expression.** Stable PDX1-eGFP human ES cells were differentiated to pancreatic progenitor stage and treated with either DMSO (controls) or Verteporfin for 5 days and immuno stained with C-peptide (white), Glucagon (dark grey) and GFP PDX1 (light grey). Verteporfin-treated progenitors show enhanced endocrine/beta cell differentiation and express higher levels of C-peptide, whereas DMSO treated cells express low levels of C-peptide and Glucagon.

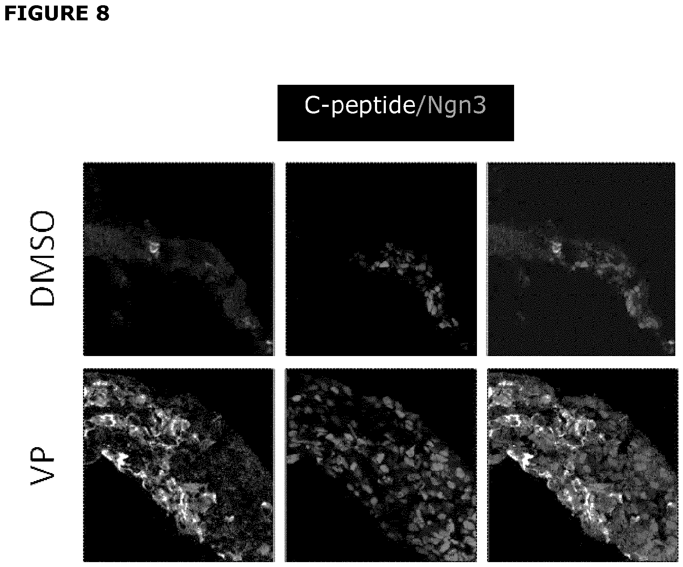

Figure 8: hES cell derived pancreatic progenitors treated with VP for 5 days **show enhanced endocrine differentiation & Insulin expression.** Stable Pdx1 Gfp human ES cells were differentiated to pancreatic progenitor stage and then treated with either DMSO (control) or Verteporfin (VP, 1 µg/ml) for 5 additional days. Cultures were immuno-stained with c-peptide (light grey) and Neurogenin3 (dark grey) antibodies and imaged using confocal microscope. Verteporfin treated cultures exhibit enhanced endocrine and beta cell differentiation and express higher levels of Ngn3 and c-peptide compared to DMSO treated cells.

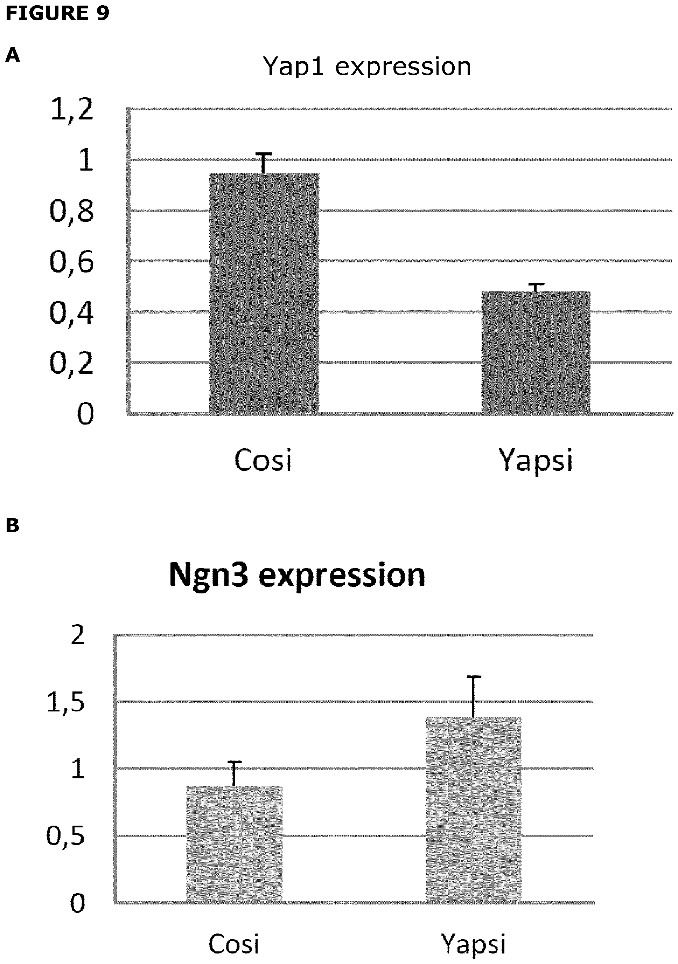

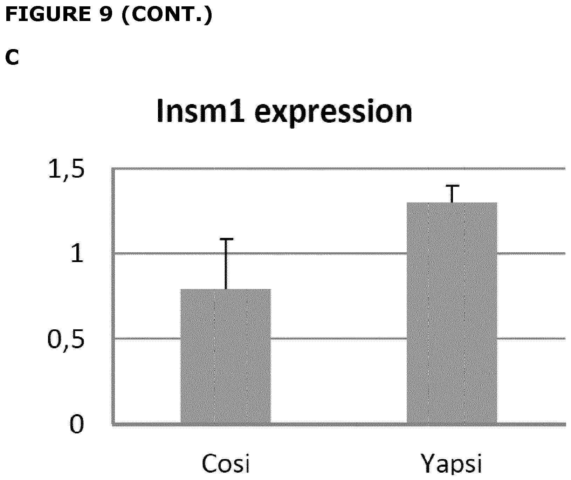

**Figure 9****: Transient loss of Yap1 in Pdx1 GFP hES cells leads to up-regulation of endocrine progenitor gene expression.** Stable Pdx1 Gfp human ES cells were differentiated to pancreatic progenitor stage and then transiently transfected with control (Cosi) or Yap1 siRNA. Cultures were analyzed using RT-qPCR after 3 days of siRNA transfection. Cells transfected with Yap1 siRNA showed 50% reduction in Yap1 mRNA levels (A) and this results in increased endocrine progenitors Ngn3 (B) and Insm1 (C) expression. Y axis: relative gene expression.

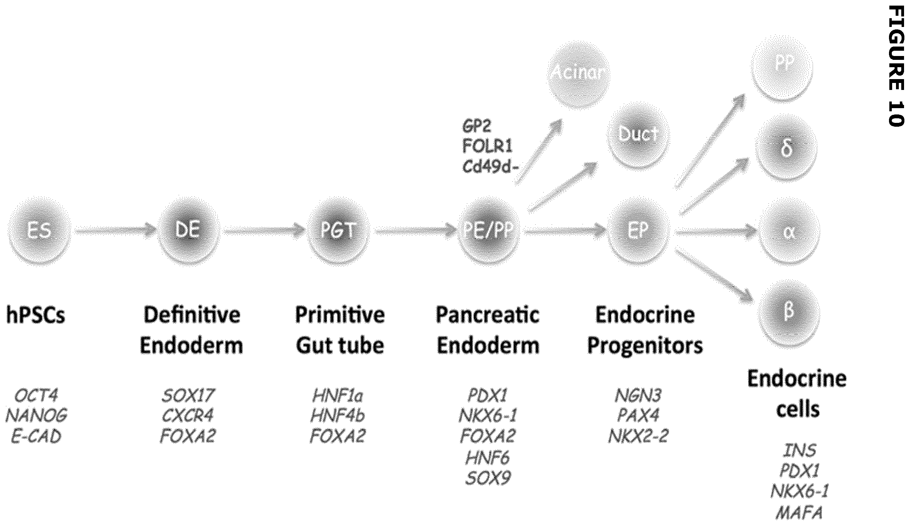

**Figure 10****: Schematic showing the intermediate stages of hESC differentiation towards insulin producing cells.** The novel cell surface markers GP2, FOLR1 and CD49d can be used to isolate pancreatic progenitor cells at PE stage. These pancreatic progenitor cells have the capacity to differentiate into acinar-, ductal- , and also the endocrine cells that comprise the pancreas.

### Detailed description

The invention is as defined in the claims. Any embodiment not falling under the scope of the appended claims does not form part of the invention.

In one aspect, the present disclosure relates to a method for differentiation of cells into insulin-producing β-cells, said method comprising the steps of:
i) providing a pancreatic progenitor cell population comprising at least one cell capable of differentiation;
ii) incubating said cell population in the presence of a Yap1 inhibitor such as verteporfin;
thereby obtaining a cell population enriched for insulin-producing β-cells.

In another aspect, the present disclosure relates to a method for differentiation of cells into insulin-producing β-cells, said method comprising the steps of:
i) inactivating Yap1 in said cells;
ii) differentiating the cells of step i) and committing them to endocrine fate, preferably to insulin producing β-cell fate;
thereby obtaining a cell population enriched for insulin-producing β-cells.

In yet another aspect, the present disclosure relates to a cell population enriched for insulin-producing β-cells obtainable by the methods described herein.

In yet another aspect, the present disclosure relates to a cell population enriched for insulin-producing β-cells obtainable by the methods described herein for treatment of a metabolic disorder in an individual in need thereof.

In yet another aspect, the present disclosure relates to a Yap1 inhibitor for use as a medicament for treating a metabolic disorder in an individual in need thereof.

In yet another aspect, the present disclosure relates to a method of treatment of a metabolic disorder in an individual in need thereof, said method comprising the steps of:
i) providing a pancreatic progenitor cell population comprising at least one cell capable of differentiation;
ii) incubating said cell population in the presence of a Yap1 inhibitor such as verteporfin, thereby obtaining a cell population enriched for insulin-producing β-cells;
iii) transplanting said cell population enriched for insulin-producing β-cells in said individual.

In yet another aspect, the disclosure relates to a method for identifying a Yap1 inhibitor, said method comprising the steps of:
i) Providing a compound;
ii) Incubating said compound in the presence of Yap1;
iii) Measuring Yap1 activity and determining whether said compound is a Yap1 inhibitor.

### Definitions

Antibody. The term 'antibody' describes a functional component of serum and is often referred to either as a collection of molecules (antibodies or immunoglobulin) or as one molecule (the antibody molecule or immunoglobulin molecule). An antibody molecule is capable of binding to or reacting with a specific antigenic determinant (the antigen or the antigenic epitope), which in turn may lead to induction of immunological effector mechanisms. An individual antibody molecule is usually regarded as monospecific, and a composition of antibody molecules may be monoclonal (i.e., consisting of identical antibody molecules) or polyclonal (i.e., consisting of different antibody molecules reacting with the same or different epitopes on the same antigen or on distinct, different antigens). Each antibody molecule has a unique structure that enables it to bind specifically to its corresponding antigen, and all natural antibody molecules have the same overall basic structure of two identical light chains and two identical heavy chains. Antibodies are also known collectively as immunoglobulins. The terms antibody or antibodies as used herein is used in the broadest sense and covers intact antibodies, chimeric, humanized, fully human and single chain antibodies, as well as binding fragments of antibodies, such as Fab, F(ab')₂, Fv fragments or scFv fragments, as well as multimeric forms such as dimeric IgA molecules or pentavalent IgM.
Antigen. An antigen is a molecule comprising at least one epitope. The antigen may for example be a polypeptide, polysaccharide, protein, lipoprotein or glycoprotein.
Bona fide pancreatic progenitor cell. The term 'bona fide pancreatic progenitor cell' or 'true pancreatic progenitor' refers herein to a cell, which is capable of differentiating into all pancreatic lineages, including acinar, duct and endocrine, such as insulin-producing cells.
Definitive endoderm. As used herein, "definitive endoderm" or "DE" refers to a multipotent cell that can differentiate into cells of the gut tube or organs derived from the gut tube. In accordance with certain embodiments of the present disclosure, the definitive endoderm cells and cells derived therefrom are mammalian cells, and in a preferred embodiment of the present disclosure, the definitive endoderm cells are human cells. In some embodiments of the present disclosure, definitive endoderm cells express or fail to significantly express certain markers. In some embodiments of the present disclosure, one or more markers selected from SOX17, CXCR4, MIXLI, GAT A4, FOXA2, GSC, FGF 17, VWF, CALCR, FOXQI, CMKORI, CER and CRIPI are expressed in definitive endoderm cells. In other embodiments of the present disclosure, one or more markers selected from OCT4, HNF4A, alpha-fetoprotein (AFP), Thrombomodulin (TM), SPARC and SOX7 are not significantly expressed in definitive endoderm cells. Definitive endoderm cells do not express PDX1.
Differentiable or differentiated cell. As used herein, the phrase, "differentiable cell" or "differentiated cell" or "hES-derived cell" can refer to pluripotent, multipotent, oligopotent or even unipotent cells, as defined in detail below. In certain embodiments of the present disclosure, the differentiable cells are pluripotent differentiable cells. In more specific embodiments of the present disclosure, the pluripotent differentiable cells are selected from the group consisting of embryonic stem cells, primitive ectoderm cells, primordial germ cells, and teratocarcinoma cells. In one particular embodiment of the present disclosure, the differentiable cells are mammalian embryonic stem cells. In a more particular embodiment of the present disclosure, the differentiable cells are human embryonic stem cells. Certain embodiments of the present disclosure also contemplate differentiable cells from any source within an animal, provided the cells are differentiable as defined herein. For example, differentiable cells can be harvested from non-human embryos, or any primordial germ layer therein, from placental or chorion tissue, or from more mature tissue such as adult stem cells including, but not limited to adipose, bone marrow, nervous tissue, mammary tissue, liver tissue, pancreas, epithelial, respiratory, gonadal and muscle tissue. In specific embodiments of the present disclosure, the differentiable cells are embryonic stem cells obtained without destruction of embryos. In other specific embodiments of the present disclosure, the differentiable cells are adult stem cells. In still other specific embodiments of the present disclosure, the stem cells are placental- or chorionic-derived stem cells. Differentiation. As used herein, the term "differentiation" refers to the production of a cell type that is more differentiated than the cell type from which it is derived. The term therefore encompasses cell types that are partially and terminally differentiated. Similarly, "produced from hESCs," "derived from hESCs," "differentiated from hESCs," "h-ES derived cell" and equivalent expressions refer to the production of a differentiated cell type from hESCs in vitro and in vivo.
Embryonic. As used herein, "embryonic" refers to a range of developmental stages of an organism beginning with a single zygote and ending with a multicellular structure that no longer comprises pluripotent or totipotent cells other than developed gametic cells.
Expression level. As used herein, the term "expression level" can refer to the level of transcript (mRNA) or to the level of protein for a particular gene or protein, respectively. Expression levels can thus be determined by methods known in the art, by determining transcription level or protein level. Transcription levels can be measured by quantifying the amount of transcript by methods such as, but not limited to, Northern blot, RT-PCR or microarray-based methods. Protein levels can be measured by methods such as, but not limited to, Western blot and immunostaining.
Human embryonic stem cells. The human embryonic stem cells are pluripotent and are able to differentiate into all derivatives of the three primary germ layers namely: ectoderm, endoderm and mesoderm (Thomson et al., 1998). As used herein, the term "human pluripotent stem cells" (hPS) refers to cells that are capable, under appropriate conditions, of producing human progeny of different cell types that are derivatives of all of the 3 germinal layers
   (endoderm, mesoderm, and ectoderm). hPS cells may have the ability to form a teratoma in 8-12 week old SCID mice and/or the ability to form identifiable cells of all three germ layers in tissue culture. Included in the definition of human pluripotent stem cells are embryonic cells of various types as well as induced pluripotent stem cells (see, e.g. Yu et al. (2007) Science 318:5858; Takahashi et al. (2007) Cell 131 (5):861). The various methods and other embodiments of the present disclosure described herein may require or utilise hPS cells (hPSCs) from a variety of sources. For example, hPS cells suitable for use may be obtained from developing embryos. Additionally or alternatively, suitable hPS cells may be obtained from established cell lines and/or human induced pluripotent stem (hiPS) cells by methods which do not require the destruction of embryos (Chung et al. 2008).
   As used herein "hiPS cells" refers to human induced pluripotent stem cells.
   As used herein, the term "blastocyst-derived stem cell" is denoted BS cell, and the human form is termed "hBS cells". In literature the cells are often referred to as embryonic stem cells, and more specifically human embryonic stem cells (hESCs). The pluripotent stem cells used in the present disclosure can among others be commercially available hBS cells or cell lines. However, it is further envisaged that any human pluripotent stem cell can be used in the present disclosure, including differentiated adult cells which are reprogrammed to pluripotent cells by e.g. the treating adult cells with certain transcription factors, such as OCT4, SOX2, NANOG, and LIN28 as disclosed in Yu, et al., 2007, Takahashi et al. 2007 and Yu et al 2009. To the extent that the pancreatic progenitor cell population of the present disclosure is derived from human embryonic stem cells, the human embryonic stem cells have been generated with processes that do not involve the use of human embryos for industrial or commercial purposes.
Inactivation: The term 'inactivation' is herein used in connection with inactivation of Yap1 function in a cell and refers to manipulations of the cell in order to obtain a reduction of Yap1 function in the cell. Yap1 can be inactivated either by use of a Yap1 inhibitor, such as verteporfin. Yap1 can also be inactivated by mutation or deletion of the YAP1 gene. Silencing Yap1, for example by using siRNAs, can also be used to inactivate Yap1, as known to the person skilled in the art. Inactivation may be transient or permanent. Inactivation may also be reversible or irreversible. For example, incubation of a cell population with a Yap1 inhibitor will typically result in transient inactivation of Yap1 for as long as the inhibitor is effective. Removing the inhibitor from the environment will generally result in alleviation of the inactivation of Yap1. Likewise, siRNAs will typically only silence Yap1 for as long as they are expressed or present. Deletion or mutation of Yap1 will typically result in permanent inactivation, although the person skilled in the art will know how to reverse the effects of deletion or mutation of Yap1, for example by gene editing methods.
Induced pluripotent stem cell. Induced pluripotent stem cells (or iPSCs) can be derived directly from adult cells by reprogramming (Takashashi et al., 2006). iPSCs can be induced by proteins and are then termed protein-induced pluripotent stem cells (piPSCs).
Ligand. As used herein, "ligand" refers to a moiety or binding partner that specifically binds or cross-reacts to the marker or target or receptor or membrane protein on the cell or to the soluble analyte in a sample or solution. The target on the cell, includes but is not limited to a marker. Examples of such ligands include, but are not limited to, an antibody that binds a cellular antigen, an antibody that binds a soluble antigen, an antigen that binds an antibody already bound to the cellular or soluble antigen; a lectin that binds to a soluble carbohydrate or to a carbohydrate moiety which is a part of a glycoprotein or glycolipid; or functional fragments of such antibodies and antigens that are capable of binding; a nucleic acid sequence sufficiently complementary to a target nucleic acid sequence of the cellular target or soluble analyte to bind the target or analyte sequence, a nucleic acid sequence sufficiently complementary to a ligand nucleic acid sequence already bound to the cellular marker or target or soluble analyte, or a chemical or proteinaceous compound, such as biotin or avidin. Ligands can be soluble or can be immobilized on the capture medium (i.e., synthetically covalently linked to a bead), as indicated by the assay format, e.g., antibody affinity chromatography. As defined herein, ligands include, but are not limited to, various agents that detect and react with one or more specific cellular markers or targets or soluble analytes.
Marker. As used herein, "marker", "epitope", "target", "receptor" or equivalents thereof can refer to any molecule that can be observed or detected. For example, a marker can include, but is not limited to, a nucleic acid, such as a transcript of a specific gene, a polypeptide product of a gene, such as a membrane protein, a non-gene product polypeptide, a glycoprotein, a carbohydrate, a glycolipid, a lipid, a lipoprotein or a small molecule (for example, molecules having a molecular weight of less than 10,000 amu). A "cell surface marker" is a marker present at the cell surface.
Multipotent cell. As used herein, "multipotent" or "multipotent cell" refers to a cell type that can give rise to a limited number of other particular cell types. Multipotent cells are committed to one or more embryonic cell fates, and thus, in contrast to pluripotent cells, cannot give rise to each of the three germ layer lineages as well as extraembryonic cells.
Naive stem cell and primed stem cell. Naïve stem cells have the potential to develop into any kind of cell, unlike primed stem cells, which are able to differentiate into several types of cells but are already predetermined to some extent. Naïve stem cells have been known to exist in mice but human naïve stem cells have only been described recently (Takashima et al., 2014). Naïve stem cells can self-renew continuously without ERK signalling, are phenotypically stable, and are karyotypically intact. They differentiate in vitro and form teratomas in vivo. Metabolism is reprogrammed with activation of mitochondrial respiration as in ESC. The pluripotent state of human cells can be reset by short-term expression of two components, NANOG and KLF2, as described in Takashima et al., 2014. Naive PSCs share many properties with the inner cell mass of the blastocyst, while the primed PSCs resemble epiblast cells of a more advanced, pregastrulating stage embryo. In the mouse, the naive state is represented by embryonic stem cells (mESCs) and the primed state by epiblast stem cells (EpiSCs). In humans, blastocyst derived ESCs have been regarded until recently as the human equivalent of mESCs. However, without being bound by theory, based on multiple characteristics such as flat morphology, dependence on growth factors, or X-chromosome inactivation, hESCs (and human induced pluripotent stem cell (hiPSCs)) are closer to mouse EpiSCs than to mESCs and, as such, more likely correspond to the primed rather than the naive state of pluripotency (Tesar et al. 2007; Stadtfeld and Hochedlinger 2010).
Naturally occurring antibody. The term 'naturally occurring antibody' refers to heterotetrameric glycoproteins capable of recognising and binding an antigen and comprising two identical heavy (H) chains and two identical light (L) chains interconnected by disulfide bonds. Each heavy chain comprises a heavy chain variable region (abbreviated herein as V_{H}) and a heavy chain constant region (abbreviated herein as C_{H}). Each light chain comprises a light chain variable region (abbreviated herein as V_{L}) and a light chain constant region (abbreviated herein as C_{L}). The V_{H} and V_{L} regions can be further subdivided into regions of hypervariability, termed complementarity determining regions (CDRs), interspersed with regions that are more conserved, termed framework regions (FRs). Antibodies may comprise several identical heterotetramers.
Pancreatic progenitor cell or multipotent pancreatic progenitor cell. A progenitor cell is a cell that is committed to differentiate into a certain type of cell. Pancreatic progenitor cells are thus multipotent and can differentiate and give rise to all cell types of the pancreas.
Partially mature cell. As used herein, "partially mature cells" refer to cells that exhibit at least one characteristic of the phenotype, such as morphology or protein expression, of a mature cell from the same organ or tissue. Some embodiments of the present disclosure contemplate using differentiable cells from any animal capable of generating differentiable cells, e.g., pancreatic type cells such as beta cells. The animals from which the differentiable cells are harvested can be vertebrate or invertebrate, mammalian or non-mammalian, human or non-human. Examples of animal sources include, but are not limited to, primates, rodents, canines, felines, equines, bovines and porcines.
Pluripotent cell. By "pluripotent" is meant that the cell can give rise to each of the three germ layer lineages. Pluripotent cells, however, may not be capable of producing an entire organism. In certain embodiments of the present disclosure, the pluripotent cells used as starting material are stem cells, including human embryonic stem cells. Pluripotent cells can be derived by explanting cells from embryos at different stages of development. PSCs (pluripotent stem cells) can be classified into two distinct states, naive and primed, depending on which stage they are during embryonic development.
Stem cell. A stem cell is an undifferentiated cell that can differentiate into specialized cells and can divide to produce more stem cells. The term stem cell comprises embryonic stem cells, adult stem cells, naïve stem cells as well as induced pluripotent stem cells. Stem cells are defined by their ability at the single cell level to both self-renew and differentiate to produce progeny cells, including self-renewing progenitors, non-renewing progenitors, and terminally differentiated cells. Stem cells are also characterized by their ability to differentiate in vitro into functional cells of various cell lineages from multiple germ layers (endoderm, mesoderm and ectoderm), as well as to give rise to tissues of multiple germ layers following transplantation and to contribute substantially to most, if not all, tissues following injection into blastocysts. Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages, but all within a particular tissue, organ, or physiological system (for example, hematopoietic stem cells (HSC) can produce progeny that include HSC (self- renewal), blood cell restricted oligopotent progenitors and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g., spermatogenic stem cells).
Totipotent stem cell: The term refers to a cell having the potential to give rise to any and all types of human cells such as all three germ layer lineages and extraembryonic lineages. It can give rise to an entire functional organism.
Yap1 inhibitor. The term will herein be used interchangeably with the terms Yap-TEAD inhibitor, YAP1-TEAD inhibitor. It refers to compounds capable of inhibiting Yap1 activity, for example by disrupting the interaction between Yap1 and TEAD transcription factors.

In the present disclosure, any gene or protein name can refer to the gene or the protein in any species. For example, PDX1 or Pdx1 are used interchangeably and can refer to either murine Pdx1 or human PDX1 or to Pdx1 in another species.

In the present disclosure, a "-" sign after a gene or protein name means that the gene or protein is not expressed, while a "+" sign after a gene or protein name means that the gene or protein is expressed. Thus PDX1- or PDX1⁻ cells are cells that do not express PDX1, while PDX1+ or PDX1⁺ cells are cells that express PDX1.

YAP1 (Yes-associated protein 1) is a downstream nuclear effector of the Hippo signalling pathway, which is involved in development, growth, repair, and homeostasis. Yap1 hyperactivation is known to play a role in the development and progression of multiple cancers and may function as a potential target for cancer treatment. Alternative splicing results in multiple transcript variants encoding different isoforms. Yap1 can act both as a co-activator and a co-repressor and plays a pivotal role in organ size control and tumor suppression by restricting proliferation and promoting apoptosis. The core of this pathway is composed of a kinase cascade wherein STK3/MST2 and STK4/MST1, in complex with its regulatory protein SAV1, phosphorylates and activates LATS1/2 in complex with its regulatory protein MOB1, which in turn phosphorylates and inactivates YAP1 onco-protein and WWTR1/TAZ. It also plays a role in controlling cell proliferation in response to cell contact. Phosphorylation of YAP1 by LATS1/2 inhibits its translocation into the nucleus to regulate cellular genes important for cell proliferation, cell death, and cell migration. The presence of TEAD transcription factors are required for it to stimulate gene expression, cell growth, anchorage-independent growth, and epithelial mesenchymal transition (EMT) induction.

Verteporfin is a small molecule which is capable of inhibiting Yap1 interaction with TEAD transcription factors, thus inhibiting Yap1 activity. This benzoporphyrin derivative is a medication used as a photosensitizer for photodynamic therapy to eliminate abnormal blood vessels.

In the pancreas several different types of pancreatic cells may be found. The pancreatic cells include for example multi-potent pancreatic progenitor cells, ductal/acinar progenitor cells, fully differentiated acinar/exocrine cells, ductal/endocrine progenitor cells, endocrine progenitor cells, early endocrine cells, and/or fully differentiated endocrine cells. The different stages of hPSCs towards endocrine cells are represented in figure 6. Pancreatic endoderm progenitor cells expressing PDX1 and NKX6-1 have the capacity to differentiate into acinar cells, ductal cells or endocrine cells. The term 'bona fide pancreatic progenitor cell' or 'true pancreatic progenitor' refers herein to a cell which is capable of differentiating into all pancreatic lineages, including acinar, duct and endocrine, such as insulin-producing cell lineages.

Pancreatic early endocrine cells are cells which have initiated expression of one of the pancreatic endocrine hormones (insulin, glucagon, somatostatin and pancreatic polypeptide) but do not share all the characteristics of fully mature pancreatic endocrine cells found in the Islet of Langerhans in the adult pancreas. These cells may be endocrine cells which have turned off Ngn3 but do not share all the characteristics of fully differentiated pancreatic endocrine cells found in the Islet of Langerhans in the adult pancreas, such as responsiveness to glucose, and are positive for one of the pancreatic endocrine hormones (insulin, glucagon, somatostatin, pancreatic polypeptide, and ghrelin).

Pancreatic endocrine cells, or pancreatic hormone-producing cells, are cells capable of expressing at least one of the following hormones: insulin, glucagon, somatostatin, pancreatic polypeptide and ghrelin.

"Pancreatic fully differentiated endocrine cells" (also termed "fully differentiated endocrine cells", "pancreatic mature endocrine cells", "pancreatic endocrine cells" or "pancreatic adult endocrine cells") are cells which share all the characteristics of fully differentiated pancreatic endocrine cells found in the Islet of Langerhans in the adult pancreas.

PDX1 (Pancreatic and duodenal homeobox 1), also known as insulin promoter factor 1, is a transcription factor necessary for pancreatic development and β-cell maturation. In embryonic development, PDX1 is expressed by a population of cells in the posterior foregut region of the definitive endoderm, and PDX1⁺ epithelial cells give rise to the developing pancreatic buds, and eventually, the whole of the pancreas-its exocrine, endocrine, and ductal cell populations. PDX1 is also necessary for β-cell maturation: developing β-cells co-express PDX1, NKX6-1, and insulin, a process that results in the silencing of MAFB and the expression of MAFA, a necessary switch in maturation of β-cells. PDX1⁺ pancreatic progenitor cells also co-express HLXB9, HNF6, PTF1a and NKX6-1 (homeobox protein Nkx-6.1), and these progenitor cells form the initial pancreatic buds, which may further proliferate. Pancreatic endocrine cells express PDX1 and NKX6-1 (PDX1+ NKX6-1+ cells).

The present disclosure is based on the surprising finding that Yap1 inhibitors can be used to induce differentiation of pancreatic progenitor cells into insulin-producing β-cells. Other methods for inactivating may also be employed, such as genetic methods, e.g. mutation, deletion or silencing.

### Method for differentiation of cells into insulin-producing β-cells

In a first aspect, the disclosure relates to a method for differentiation of cells into insulin-producing β-cells, said method comprising the steps of:
i) providing a pancreatic progenitor cell population comprising at least one cell capable of differentiation;
ii) incubating said cell population in the presence of a Yap1 inhibitor such as verteporfin;
thereby obtaining a cell population enriched for insulin-producing β-cells.

In some embodiments of the present disclosure, the pancreatic progenitor cell population provided in step i) is enriched for bona fide pancreatic progenitor cells. In some embodiments of the present disclosure, the bona fide pancreatic progenitor cells express Pdx1. In some embodiments of the present disclosure, the bona fide pancreatic progenitor cells express Pdx1 and Nkx6-1.

### Starting cell population

The term 'starting cell population' herein refers to the pancreatic progenitor cell population provided in step i) comprising at least one cell capable of differentiation.

In some embodiments of the present disclosure, the cell population may be derived or isolated from an individual, such as, but not limited to, a mammal, for example a human.

In some embodiments of the present disclosure, the cells are derived from cells capable of differentiation, such as pluripotent stem cells, for example human pluripotent stem cells (hPSCs). hPSCs include human induced pluripotent stem cells (hiPSCs), human embryonic stem cells (hESCs) and naïve human stem cells (NhSCs).

In one embodiment of the present disclosure, the starting cell population is obtained from a pancreas, including a foetal pancreas or an adult pancreas. In one aspect of the present disclosure, the pancreas is from a mammal, such as a human.

In another embodiment of the present disclosure, the starting cell population is a somatic cell population. In some embodiments of the present disclosure, the starting cell population is obtained from a somatic cell population. In a further aspect of the present disclosure, the somatic cell population has been induced to de-differentiate into an embryonic-like stem cell (ESC, e.g. a pluripotent cell, or hESCs for human ESCs). Such dedifferentiated cells are also termed induced pluri-potent stem cells (IPSCs, or hIPSCs for human IPSCs).

In yet another embodiment of the present disclosure, the starting cell population is ESCs or hESCs. In one embodiment of the present disclosure, the starting cell population is obtained from ESCs or hESCs. In some embodiments of the present disclosure, the starting cell population is a population of pluripotent cells such as ESC like-cells.

In some embodiments of the present disclosure, the starting cell population comprises at least some cells capable of maturing into bona fide β-cells.

In one aspect of the disclosure, the starting cell population is of mammalian origin. In some aspects of the disclosure, the cell population has been differentiated to the pancreatic endocrine lineage.

In one aspect of the disclosure, the starting cell population is obtained from one or more donated pancreases. The methods described herein are not dependent on the age of the donated pancreas. Accordingly, pancreatic material isolated from donors ranging in age from embryos to adults can be used.

Once a pancreas is harvested from a donor, it is typically processed to yield individual cells or small groups of cells for culturing using a variety of methods. One such method calls for the harvested pancreatic tissue to be cleaned and prepared for enzymatic digestion. Enzymatic processing is used to digest the connective tissue so that the parenchyma of the harvested tissue is dissociated into smaller units of pancreatic cellular material. The harvested pancreatic tissue is treated with one or more enzymes to separate pancreatic cellular material, substructures, and individual pancreatic cells from the overall structure of the harvested organ. Collagenase, DNAse, Lipase preparations and other enzymes are contemplated for use with the methods disclosed herein.

Isolated source material can be further processed to enrich for one or more desired cell populations prior to performing the present methods. In some aspects of the present disclosure unfractionated pancreatic tissue, once dissociated for culture, can also be used directly in the culture methods of the present disclosure without further separation. However, unfractionated pancreatic tissue, once dissociated for culture, can also be used directly in the culture methods of the present disclosure without further separation, and will yield the intermediate cell population. In one embodiment of the present disclosure the isolated pancreatic cellular material is purified by centrifugation through a density gradient (e. g., Nycodenz, Ficoll, or Percoll). The mixture of cells harvested from the donor source will typically be heterogeneous and thus contain alpha cells, beta cells, delta cells, ductal cells, acinar cells, facultative progenitor cells, and other pancreatic cell types.

A typical purification procedure results in the separation of the isolated cellular material into a number of layers or interfaces. Typically, two interfaces are formed. The upper interface is islet-enriched and typically contains 10 to 100% islet cells in suspension.

The second interface is typically a mixed population of cells containing islets, acinar, and ductal cells. The bottom layer is the pellet, which is formed at the bottom of the gradient. This layer typically contains primarily acinar cells, some entrapped islets, and some ductal cells. Ductal tree components can be collected separately for further manipulation.

The cellular constituency of the fractions selected for further manipulation will vary depending on which fraction of the gradient is selected and the final results of each isolation. When islet cells are the desired cell type, a suitably enriched population of islet cells within an isolated fraction will contain at least 10% to 100 % islet cells. Other pancreatic cell types and concentrations can also be harvested following enrichment. For example, the culture methods described herein can be used with cells isolated from the second interface, from the pellet, or from other fractions, depending on the purification gradient used.

In one embodiment of the present disclosure, intermediate pancreatic cell cultures are generated from the islet-enriched (upper) fraction. Additionally, however, the more heterogeneous second interface and the bottom layer fractions that typically contain mixed cell populations of islets, acinar, and ductal cells or ductal tree components, acinar cells, and some entrapped islet cells, respectively, can also be used in culture. While both layers contain cells capable of giving rise to the enriched bona fide pancreatic progenitor cell population described herein, each layer may have particular advantages for use with the disclosed methods.

In one embodiment of the present disclosure, the starting cell population is a population of or comprising stem cells. In another embodiment of the present disclosure, the starting cell population is a population of or comprising stem cells differentiated to the pancreatic endocrine lineage. In one embodiment of the present disclosure, the cell population is a population of stem cells that is obtained without the destruction of an embryo. Methods for obtaining stem cells without destroying embryos are known in the art (Chung et al., 2008).

A protocol for obtaining pancreatic cells from stem cells is exemplified by, but not limited to, the protocols described in D'Amour, K. A. et al. (2006); Jiang, J. et al. (2007); and Kroon, E. et al. (2008), Rezania et al (2012, 2014), Felicia W. Pagliuca et al (2014).

A protocol for obtaining pancreatic cells from somatic cells or somatic cells induced to dedifferentiate into pluripotent cells such as ES like-cells is exemplified by, but not limited to, the protocols described in Aoi, T. et al. (2008), D'Amour, K. A. et al. (2006), Jiang, J. et al. (2007), Kroon, E. et al. (2008), Takahashi, K. et al. (2007), Takahashi and Yamanaka (2006), and Wernig, M. et al. (2007).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

In some aspects of the present disclosure "differentiate" or "differentiation" as used herein refers to a process where cells progress from an immature state to a less immature state. In another aspect of the present disclosure "differentiate" or "differentiation" as used herein refers to a process where cells progress from an undifferentiated state to a differentiated state or from an immature state to a mature state. For example, undifferentiated pancreatic cells may be able to proliferate and express characteristics markers, like PDX1. Early undifferentiated embryonic pancreatic cells may be able to proliferate and express characteristics markers, like PDX1. In one embodiment of the present disclosure mature or differentiated pancreatic cells do not proliferate and secrete high levels of pancreatic endocrine hormones. In some embodiments of the present disclosure mature or differentiated pancreatic cells do not proliferate and secrete high levels of pancreatic endocrine hormones or digestive enzymes. In one embodiment of the present disclosure, e.g., mature beta cells secrete insulin at high levels. In some embodiments of the present disclosure e.g., mature beta cells secrete insulin at high levels in response to glucose. Changes in cell interaction and maturation occur as cells lose markers of undifferentiated cells or gain markers of differentiated cells. In one embodiment of the present disclosure loss or gain of a single marker can indicate that a cell has "matured or differentiated". In some embodiments of the present disclosure loss or gain of a single marker can indicate that a cell has "matured or fully differentiated". The term "differentiation factors" refers to a compound added to pancreatic cells to enhance their differentiation to mature endocrine cells also containing insulin producing beta cells. Exemplary differentiation factors include hepatocyte growth factor, keratinocyte growth factor, exendin-4, basic fibroblast growth factor, insulin-like growth factor-I, nerve growth factor, epidermal growth factor platelet-derived growth factor, and glucagon-like-peptide 1. In one embodiment of the present disclosure, differentiation of the cells comprises culturing the cells in a medium comprising one or more differentiation factors.

In some embodiments of the present disclosure, the starting cell population is analysed to identify whether at least one of the cells of the starting population expresses markers characteristic of the pancreatic endocrine lineage and selected from the group consisting of NGN3, NEUROD, ISL1, PDX1, NKX6.1, NKX2.2, MAFA, MAFB, ARX, BRN4, PAX4 and PAX6, GLUT2, INS, GCG, SST, pancreatic polypeptide (PP). In some embodiments of the present disclosure markers characteristic of the pancreatic endocrine lineage are selected from the group consisting of PDX1 and NKX6-1. In one embodiment of the present disclosure, a pancreatic endocrine cell is capable of expressing at least one of the following hormones: insulin, glucagon, somatostatin, and PP. In some embodiments of the present disclosure, a pancreatic endocrine cell is capable of expressing at least one of the following hormones: insulin, glucagon, somatostatin, PP and ghrelin. Suitable for use in the present disclosureis a starting cell population comprising at least one cell that expresses at least one of the markers characteristic of the pancreatic endocrine lineage. In one aspect of the present disclosure, a cell expressing markers characteristic of the pancreatic endocrine lineage is a pancreatic endocrine cell. The pancreatic endocrine cell may be a pancreatic hormone expressing cell. Alternatively, the pancreatic endocrine cell may be a pancreatic hormone secreting cell.

In one embodiment of the present disclosure, the pancreatic endocrine cell is a cell expressing markers characteristic of the beta cell lineage. A cell expressing markers characteristic of the beta cell lineage expresses PDX1 and at least one of the following transcription factors: NGN-3, NKX2-2, NKX6-1, NeuroD, Isl-1, Hnf-3 beta, MAFA, PAX-4, Insm1, Arx and PAX-6. In one embodiment of the present disclosure, a cell expressing markers characteristic of the beta cell lineage is a beta cell. In one embodiment of the present disclosure, the pancreatic endocrine cell is a cell expressing the marker NKX6-1. In another aspect of the present disclosure, the pancreatic endocrine cell is a cell expressing the marker PDX1. In a further aspect of the present disclosure, the pancreatic endocrine cell is a cell expressing the markers NKX6-1 and PDX1.

PDX1 is homeodomain transcription factor implicated in pancreas development. PAX-4 is a beta cell specific factor and PAX-6 is a pancreatic islet cell (specific) transcription factor; both are implicated in islet development. HNF-3 beta (also known as FOXA2) belongs to the hepatic nuclear factor family of transcription factors, which is characterized by a highly conserved DNA binding domain and two short carboxy-terminal domains. NeuroD is basic helix-loop-helix (bHLH) transcription factor implicated in neurogenesis. NGN3 is a member of the neurogenin family of basic loop-helix-loop transcription factors. NKX2-2 and NKX6-1 as used herein are members of the Nkx transcription factor family. Islet-1 or ISL-1 is a member of the LIM/homeodomain family of transcription factors, and is expressed in the developing pancreas. MAFA is a transcription factor expressed in the pancreas, and controls the expression of genes involved in insulin biosynthesis and secretion. INSM1 encodes the insulinoma-associated protein 1. INSM1 represents an important player in early embryonic neurogenesis. In pancreatic endocrine cell differentiation, Ngn3 first activates INSM1 and subsequently NeuroD/β2. Conversely, INSM1 exerts a feedback mechanism to suppress NeuroD/β2 and its own gene expression. INSM1 gene ablation in the mouse results in the impairment of pancreatic endocrine cell maturation. Further, deletion of INSM1 severely impairs catecholamine biosynthesis and secretion from the adrenal gland that results in early embryonic lethality. Arx encodes Aristaless related homeobox, which is involved in central nervous system and pancreas development. Mutations of Arx in mice are associated with hypoglycemia. Arx exhibits Ngn3-dependent expression throughout endocrine pancreas development in α, β-precursor, and δ cells.

NKX6.1 and PDX1 are co-expressed with PTF1a in the early pancreatic multipotent cell that can develop into all cell types found in the adult pancreas (e.g., acinar, ductal, and endocrine cells). Within this cell population cells that also transiently express Ngn3 are found. Once a cell express or has expressed NGN3 it will be part of the endocrine lineage, giving rise to endocrine cells (one type being the insulin producing beta cell) that will later form the Islets of Langerhans. In the absence of NGN3 no endocrine cells form during pancreas development. As development progress NKX6-1 and PDX1 are co-expressed in the more central domain of the pancreas, which now becomes devoid of PTF1a expression and the NKX6-1 and PDX1 positive cells can no longer give rise to acinar cells. Within this NKX6-1 and PDX1 positive cell population a significant number of cells transiently co-express NGN3, marking them for the endocrine lineage like earlier in development.

In one embodiment of the present disclosure, the starting cell population is derived from cells capable of differentiation. In a specific embodiment of the present disclosure, the at least one cell capable of differentiation is a human pluripotent stem cell. In some embodiments of the present disclosure, the at least one cell capable of differentiation is selected from the group consisting of human iPS cells (hIPSCs), human ES cells (hESCs) and naive human stem cells (NhSCs).

The cells capable of differentiation may be derived from cells isolated from an individual.

In one embodiment of the present disclosure, at least one cell of the starting cell population has the capability to differentiate further. It may have the capability to differentiate further into pancreatic hormone-producing cells. In some embodiments of the present disclosure, at least one of the pancreatic hormone-producing cells is an insulin-producing cell. In some embodiments of the present disclosure, at least one of the pancreatic hormone-producing cells is responsive to glucose. In some embodiments of the present disclosure, at least one of the pancreatic hormone-producing cells is an insulin-producing cell which is also responsive to glucose. In some embodiments of the present disclosure, at least one cell of the starting cell population can produce insulin-producing islet cells.

In some embodiments of the present disclosure, at least one of the pancreatic hormone-producing cells is also capable of expressing MAFA. In one embodiment of the present disclosure, the pancreatic hormone-producing cell is a mature β cell.

Methods for obtaining pancreatic endoderm cells from definitive endoderm cells are known to the person skilled in the art. An example of such a method is given in example 4. Briefly, definitive endoderm cells can be differentiated into primitive gut tube cells, then into posterior foregut cells, and finally into pancreatic endoderm cells. The skilled person knows how to perform these steps. The step of differentiating the pancreatic endoderm cells into mature β cells can be performed as disclosed herein, for example by inhibiting Yap1 with an inhibitor such as verteporfin, or by silencing, mutating or deleting Yap1.

In some embodiments of the present disclosure, the starting cell population comprises at least one bona fide pancreatic progenitor cell, preferably expressing PDX1 and NKX6-1. The starting cell population may in some embodiments of the present disclosure be enriched for PDX1+ NKX6-1+ progenitor cells. Methods for obtaining a cell population enriched for PDX1+ NKX6-1+ progenitor cells are provided in the co-pending patent application "Isolation of bona fide pancreatic progenitor cells" (inventors: Jacqueline Ameri and Henrik Semb, applicant: University of Copenhagen) filed on the same date.

Accordingly, in some embodiments of the present disclosure the starting cell population is enriched for PDX1+ NKX6-1+ progenitor cells and is obtainable by a method comprising the steps of:
i) providing a cell population comprising at least one bona fide pancreatic progenitor cell, wherein the bona fide pancreatic progenitor cell expresses PDX1 and NKX6-1;
ii) exposing said cell population to:
   a) a first ligand which binds to a first marker specific for PDX1⁻ cells and selecting the cells that do not bind to said first ligand from said cell population, thereby enriching the cell population for PDX1⁺ cells;
      and/or
   b) a second ligand which binds to a second marker specific for PDX1⁺ cells and selecting the cells that bind to said second ligand from the cells that do not bind to said second ligand, thereby enriching the cell population for PDX1⁺ cells;
      and/or
   c) a third ligand which binds to a third marker specific for PDX1⁺ NKX6-1⁺ cells and selecting the cells that bind to said third ligand from the cells that do not bind to said third ligand, thereby enriching the cell population for PDX1⁺ NKX6-1⁺ cells;
   thereby obtaining a cell population enriched for bona fide pancreatic progenitor cells..

In particular embodiments of the present disclosure, the first ligand recognises and binds to CD49d, the second ligand recognises and binds to FOLR1, and the third ligand recognises and binds to GP2.

Such methods as described in said co-pending patent application are useful for obtaining a starting cell population comprising at least 70% bona fide pancreatic progenitor cells, such as at least 75% bona fide pancreatic progenitor cells, such as at least 80% bona fide pancreatic progenitor cells, such as at least 85% bona fide pancreatic progenitor cells, such as at least 90% bona fide pancreatic progenitor cells.

Thus in some embodiments of the present disclosure, the starting cell population enriched for bona fide pancreatic progenitor cells comprises at least 70% bona fide pancreatic progenitor cells, such as at least 71% bona fide pancreatic progenitor cells, such as at least 72% bona fide pancreatic progenitor cells, such as at least 73% bona fide pancreatic progenitor cells, such as at least 74% bona fide pancreatic progenitor cells, such as at least 75% bona fide pancreatic progenitor cells, such as at least 76% bona fide pancreatic progenitor cells, such as at least 77% bona fide pancreatic progenitor cells, such as at least 78% bona fide pancreatic progenitor cells, such as at least 79% bona fide pancreatic progenitor cells, such as at least 80% bona fide pancreatic progenitor cells, such as at least 81% bona fide pancreatic progenitor cells, such as at least 82% bona fide pancreatic progenitor cells, such as at least 83% bona fide pancreatic progenitor cells, such as at least 84% bona fide pancreatic progenitor cells, such as at least 85% bona fide pancreatic progenitor cells, such as at least 86% bona fide pancreatic progenitor cells, such as at least 87% bona fide pancreatic progenitor cells, such as at least 88% bona fide pancreatic progenitor cells, such as at least 89% bona fide pancreatic progenitor cells, such as at least 90% bona fide pancreatic progenitor cells.

In order to determine the fraction of bona fide progenitor cells comprised in the starting cell population, methods known in the art can be employed, such as, but not limited to, immunostaining and flow cytometry methods.

### Yap 1 inhibitor

YAP1 (Yes-associated protein 1) is a downstream nuclear effector of the Hippo signalling pathway which is involved in development, growth, repair, and homeostasis. Verteporfin is a small molecule which is capable of inhibiting Yap1 activity.

After a pancreatic progenitor cell population comprising at least one cell capable of differentiation has been provided as described above, the cell population is incubated in the presence of a Yap1 inhibitor. In some embodiments of the present disclosure, the Yap1 inhibitor is verteporfin.

In some embodiments of the present disclosure, the cell population is incubated in the presence of verteporfin at a concentration of between 0.1 and 10 µg/mL, such as between 0.2 and 9 µg/mL, such as between 0.3 and 8 µg/mL, such as between 0.4 and 7 µg/mL, such as between 0.5 and 6 µg/mL, such as between 0.6 and 5 µg/mL, such as between 0.7 and 4 µg/mL, such as between 0.8 and 3 µg/mL, such as between 0.9 and 2 µg/mL, such as 1 µg/mL. In one embodiment of the present disclosure, the cell population is incubated in the presence of 1 µg/mL verteporfin.

In some embodiments of the present disclosure, the cell population is incubated in the presence of verteporfin for a duration of at least 3 days, such as at least 4 days, such as at least 5 days, such as at least 6 days, such as at least 7 days, such as at least 8 days, such as at least 9 days, such as at least 10 days. In some embodiments of the present disclosure, the incubation in the presence of verteporfin is performed for a duration of 5 days.

### Population enriched for insulin-producing β-cells

The present methods comprising the steps of providing a pancreatic progenitor cell population comprising at least one cell capable of differentiation and of incubating said cell population in which Yap1 has been inactivated, for example by incubating the cells in the presence of a Yap1 inhibitor such as verteporfin or by inactivating Yap1 by genetic means as described below, can be used to obtain a cell population enriched for insulin-producing β-cells, also termed 'enriched cell population' hereinafter.

In some embodiments of the present disclosure, at least some of the cells of the enriched cell population are capable of maturing into bona fide β-cells. In other embodiments of the present disclosure, at least some of the cells of the enriched cell population are capable of maturing into bona fide α-cells.

The inactivation of Yap1, for example by the addition of a Yap1 inhibitor such as verteporfin or by inactivation by genetic means, induces differentiation of cells into insulin-producing β-cells, where the insulin-producing β-cells have increased expression of at least one marker selected from the group consisting of C-peptide, Arx, Ngn3, Insm-1, Isl-1, MafA and MafB compared to cells where Yap1 has not been inactivated. In some embodiments of the present disclosure, the cells have increased expression of at least one of Ngn3, Arx, Insm-1, Isl-1, MafA and MafB. In some embodiments of the present disclosure, the cells have increased expression of at least MafA. In some embodiments of the present disclosure, the cells have increased expression of at least Ngn3. Accordingly, the cells preferably have increased endocrine differentiation compared to cells that were incubated in the absence of Yap1 inhibitor. In some embodiments of the present disclosure, inactivation of Yap1 results in increased differentiation towards alpha pancreatic cells. In other embodiments of the present disclosure, inactivation of Yap1 results in increased differentiation towards beta pancreatic cells. In some embodiments of the present disclosure, inactivation of Yap1 results in increased differentiation towards alpha and beta pancreatic cells.

The term 'expression' or expression level' shall herein refer to either mRNA (transcript) level or to protein levels.

Methods for determining whether the insulin-producing β-cells have increased expression of a marker such as C-peptide, Ngn3, Insm-1, Isl-1, MafA or MafB are available to the skilled person. Expression levels can be measured at the mRNA level or at the protein level. Methods for measuring mRNA levels are known in the art and include, but are not limited to: real-time PCR analysis, RT-qPCR, Northern blotting and hybridization microarrays. Methods for measuring protein expression levels are also known to the skilled person and include, but are not limited to: immunostaining methods, Western Blotting, protein immunoprecipitation and immunoelectrophoresis.

In some embodiments of the present disclosure, the enriched cell population has an increased expression of glucagon by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold, such as at least 3.5-fold, such as at least 4.0-fold, such as at least 4.5-fold, such as at least 5.0-fold, such as at least 5.5-fold, such as at least 6.0-fold, such as at least 7.0-fold, such as at least 8.0-fold, such as at least 9.0-fold, such as at least 10-fold.

In some embodiments of the present disclosure, the expression of glucagon in the enriched cell population is essentially unchanged compared to cells that were incubated in cells where Yap1 was not inactivated, for example in the absence of Yap1 inhibitor. In other embodiments of the present disclosure, the expression of glucagon in the enriched cell population is decreased compared to the expression in cells where Yap1 was not inactivated, for example in cells that were incubated in the absence of Yap1 inhibitor.

In some embodiments of the present disclosure, the enriched cell population has an increased expression of Isl-1 by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold, such as at least 3.1-fold, such as at least 3.2-fold, such as at least 3.3-fold, such as at least 3.4-fold, such as at least 3.5-fold.

In some embodiments of the present disclosure, the enriched cell population has an increased expression of MafA by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold. In some embodiments of the present disclosure, the enriched cell population has an increased expression of MafA by at least 10-fold, such as at least 50-fold, such as at least 100-fold, such as at least 150-fold, such as at least 200-fold, such as at least 250-fold, such as at least 300-fold, such as at least 350-fold, such as at least 400-fold, such as at least 450-fold, such as at least 500-fold, such as at least 550-fold, such as at least 600-fold, such as at least 650-fold, such as at least 700-fold, such as at least 750-fold, such as at least 800-fold, such as at least 850-fold, such as at least 900-fold, such as at least 950-fold, such as at least 1000-fold.

In some embodiments of the present disclosure, the enriched cell population has an increased expression of MafB by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold, such as at least 3.1-fold, such as at least 3.2-fold, such as at least 3.3-fold, such as at least 3.4-fold, such as at least 3.5-fold, such as at least 3.6-fold, such as at least 3.7-fold, such as at least 3.8-fold, such as at least 3.9-fold, such as at least 4.0-fold, such as at least 4.5-fold, such as at least 5.0-fold, such as at least 6.0-fold, such as at least 6.5-fold, such as at least 7.0-fold, such as at least 7.5-fold, such as at least 8.0-fold.

In some embodiments of the present disclosure, the enriched cell population has an increased expression of Insm-1, where the expression of Insm-1 is increased by at least 1.1-fold, such as at least 1.2-fold, such as at least 1.3-fold, such as at least 1.4-fold, such as at least 1.5-fold, such as at least 2-fold, such as at least 3-fold, such as at least 4-fold, such as at least 5-fold, such as at least 6-fold, such as at least 7-fold, such as at least 8-fold, such as at least 9-fold, such as at least 10-fold, such as at least 20-fold, such as at least 30-fold, such as at least 40-fold, such as at least 50-fold, such as at least 60-fold, such as at least 70-fold, such as at least 80-fold, such as at least 90-fold, such as at least 100-fold, such as at least 120-fold, such as at least 130-fold, such as at least 140-fold, such as at least 150-fold, such as at least 160-fold, such as at least 170-fold, such as at least 180-fold, such as at least 190-fold, such as at least 200-fold, such as at least 210-fold, such as at least 220-fold.

In some embodiments of the present disclosure, the enriched cell population has an increased expression of Ngn3, where the expression of Ngn3 is increased by at least 1.1-fold, such as at least 1.2-fold, such as at least 1.3-fold, such as at least 1.4-fold, such as at least 1.5-fold, such as at least 2-fold, such as at least 3-fold, such as at least 4-fold, such as at least 5-fold, such as at least 6-fold, such as at least 7-fold, such as at least 8-fold, such as at least 9-fold, such as at least 10-fold, such as at least 15-fold, such as at least 20-fold.

In some embodiments of the present disclosure, the enriched cell population has an increased expression of NeuroD by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold, such as at least 3.1-fold, such as at least 3.2-fold, such as at least 3.3-fold, such as at least 3.4-fold, such as at least 3.5-fold, such as at least 4-fold, such as at least 5-fold, such as at least 6-fold.

In some embodiments of the present disclosure, the enriched cell population has an increased expression of Arx by at least 1.5-fold, such as at least 1.6-fold, such as at least 1.7-fold, such as at least 1.8-fold, such as at least 1.9-fold, such as at least 2-fold, such as at least 2.1-fold, such as at least 2.2-fold, such as at least 2.3-fold, such as at least 2.4-fold, such as at least 2.5-fold, such as at least 2.6-fold, such as at least 2.7-fold, such as at least 2.8-fold, such as at least 2.9-fold, such as at least 3.0-fold, such as at least 3.5-fold, such as at least 4.0-fold, such as at least 4.5-fold, such as at least 5.0-fold.

In some embodiments of the present disclosure, the enriched cell population is capable of producing more insulin than a cell population incubated in the absence of a Yap1 inhibitor such as verteporfin. Methods to determine how much insulin a cell population produces are available to the skilled person. For example, insulin can be measured at the protein level with an ELISA assay, or at the mRNA level using methods known in the art such as, but not limited to: real-time PCR analysis, RT-qPCR, Northern blotting and hybridization microarrays.

Accordingly, in some embodiments of the present disclosure the enriched cell population is capable of producing at least 1.5-fold more insulin than cells obtained when the cell population of step i) is incubated in the absence of Yap1 inhibitor, such as at least 1.6-fold more insulin, such as at least 1.7-fold more insulin, such as at least 1.8-fold more insulin, such as at least 1.8-fold more insulin, such as at least 1.9-fold more insulin, such as at least 2.0-fold more insulin, such as at least 2.5-fold more insulin, such as at least 3.0-fold more insulin, such as at least 3.5-fold more insulin, such as at least 4.0-fold more insulin, such as at least 4.5-fold more insulin, such as at least 5.0-fold more insulin, such as at least 5.5-fold more insulin, such as at least 6.0-fold more insulin, such as at least 6.5-fold more insulin, such as at least 7.0-fold more insulin, such as at least 7.5-fold more insulin, such as at least 8.0-fold more insulin, such as at least 8.5-fold more insulin, such as at least 9.0-fold more insulin, such as at least 9.5-fold more insulin, such as at least 10-fold more insulin, such as at least 12.5-fold more insulin, such as at least 15-fold more insulin, such as at least 17.5-fold more insulin, such as at least 20-fold more insulin, such as at least 25-fold more insulin, such as at least than cells incubated in the absence of Yap1 inhibitor.

In some embodiments of the present disclosure, the levels of insulin and/or of insulin transcript in the enriched cell population are increased at least 1.5-fold compared to the levels observed in a cell population incubated in the absence of Yap1 proliferation inhibitor, such as at least 1.6-fold, such as at least 1.7-fold, such as at least 1.8-fold, such as at least 1.8-fold, such as at least 1.9-fold, such as at least 2.0-fold, such as at least 2.5-fold, such as at least 3.0-fold, such as at least 3.5-fold, such as at least 4.0-fold.

The capacity of a cell population to produce insulin can also be measured by determining the insulin area of the cell population. Insulin area is the ratio between the number of insulin expressing cells and the total number of cells. The number of insulin producing cells can be determined visually by immunostaining methods, while the total number of cells can be determined visually using markers such as DAPI. The insulin cell area may be calculated from serial sections spanning pancreatic tissue stained for Insulin and DAPI (nuclei). The mean values of cross sectional expression areas from the acquired confocal images may be determined using appropriate software to quantify insulin cell area.

Accordingly, the present method can be used to obtain an enriched cell population, wherein the insulin area in said enriched cell population is increased at least 1.5-fold compared to the insulin area in cells incubated in the absence of Yap1 proliferation inhibitor, such as at least 1.6-fold, such as at least 1.7-fold, such as at least 1.8-fold, such as at least 1.8-fold, such as at least 1.9-fold, such as at least 2.0-fold, such as at least 2.5-fold, such as at least 3.0-fold, such as at least 3.5-fold, such as at least 4.0-fold.

Without being bound by theory, the increased expression levels of insulin, C-peptide, Insm-1, Isl-1, MafA or MafB observed in the enriched cell population is believed to be a consequence of an increased proportion of insulin-producing β-cells in said population compared to the proportion of insulin-producing β-cells in a cell population incubated in the absence of Yap1 inhibitor.

Accordingly, in some embodiments of the present disclosure the enriched cell population comprises at least 5% insulin-producing β-cells, such as at least 10% insulin-producing β-cells, such as at least 15% insulin-producing β-cells, such as at least 20% insulin-producing β-cells, such as at least 25% insulin-producing β-cells, such as at least 30% insulin-producing β-cells, such as at least 35% insulin-producing β-cells, such as at least 40% insulin-producing β-cells, such as at least 45% insulin-producing β-cells, such as at least 50% insulin-producing β-cells, such as at least 55% insulin-producing β-cells, such as at least 60% insulin-producing β-cells, such as at least 65% insulin-producing β-cells, such as at least 70% insulin-producing β-cells, such as at least 75% insulin-producing β-cells, or more.

### Inactivation of Yap1

It will be understood that although the methods described above relate to methods of differentiation of cells into insulin-producing β-cells by using a Yap1 inhibitor, the present disclosurecan be adapted to inactivate Yap1 directly in the cells, for example by gene editing methods.

Accordingly, in one aspect the present disclosurerelates to a method for differentiation of cells into insulin-producing β-cells, said method comprising the steps of:
i) inactivating Yap1 in said cells;
ii) differentiating the cells of step i) and committing them to endocrine fate, preferably to insulin producing β-cell fate;
thereby obtaining a cell population enriched for insulin-producing β-cells.

More specifically, a method for differentiation of cells into insulin-producing β-cells is provided, said method comprising the steps of:
i) providing a pancreatic progenitor cell population comprising at least one cell capable of differentiation;
ii) inactivating Yap1 in said cell population;
iii) differentiating the cells of step ii) and committing them to endocrine fate, preferably to insulin producing β-cell fate;
thereby obtaining a cell population enriched for insulin-producing β-cells.

Yap1 can be inactivated in said cells by using a Yap1 inhibitor as described above. Alternatively, Yap1 can be inactivated in said cells by methods such as gene editing methods or silencing methods.

In some embodiments of the present disclosure, inactivation of Yap1 results in a reduction of Yap1 expression by at least 10%, such as at least 20%, such as at least 30%, such as at least 40%, such as at least 50%, such as at least 60%, such as at least 70%, such as at least 80%, such as at least 90%, such as at least 95%, such as 100%. In some embodiments of the present disclosure, inactivation of Yap1 results in a reduction of Yap1 function by at least 10%, such as at least 20%, such as at least 30%, such as at least 40%, such as at least 50%, such as at least 60%, such as at least 70%, such as at least 80%, such as at least 90%, such as at least 95%, such as 100%.

The cells that are to be differentiated can be derived from an organism such as a mammal. In some embodiments of the present disclosure, the mammal is a mouse or a rat. In other embodiments of the present disclosure, the mammal is a human. In some embodiments of the present disclosure, cells are isolated from the organism as described above and Yap1 is inactivated, for example by mutating or deleting the Yap1 gene conditionally in the developing pancreas using Cre - flox mediated recombination or by introducing silencing means such as siRNA into the cells.

### Cell population enriched for insulin-producing β-cells

In one aspect, the present disclosure relates to a cell population enriched for insulin-producing β-cells obtainable by the methods described herein.

Accordingly, disclosed herein is a cell population comprising at least 5% insulin-producing β-cells, such as at least 10% insulin-producing β-cells, such as at least 15% insulin-producing β-cells, such as at least 20% insulin-producing β-cells, such as at least 25% insulin-producing β-cells, such as at least 30% insulin-producing β-cells, such as at least 35% insulin-producing β-cells, such as at least 40% insulin-producing β-cells, such as at least 45% insulin-producing β-cells, such as at least 50% insulin-producing β-cells, such as at least 55% insulin-producing β-cells, such as at least 60% insulin-producing β-cells, such as at least 65% insulin-producing β-cells, such as at least 70% insulin-producing β-cells, such as at least 75% insulin-producing β-cells, or more.

### Treatment of a metabolic disorder

Also disclosed herein is a cell population enriched for insulin-producing β-cells obtainable by any of the methods disclosed herein, for treatment of a metabolic disorder in an individual in need thereof.

The enriched cell populations obtainable by the methods described herein can be used for treatment of metabolic disorders. These enriched cell populations can be stored prior to use, or they can be used immediately. Once a cell population with the desired characteristics is obtained, the cells may be transplanted into an individual in need thereof. As an example, such cell-based therapy is useful for transplanting insulin-producing β-cells in individuals suffering from diabetes, whereby insulin production may be restored *in vivo.* If the starting cell population is derived from the patient him/herself, the risks of adverse immune reactions such as rejection of the transplanted cells may be reduced.

The term 'metabolic disorder' as used herein shall be construed to refer to endocrine, nutritional and metabolic diseases. Preferably, the disorder is related to a pancreatic disorder. Examples of metabolic disorders are: diabetes mellitus, including type 1 and type 2 diabetes.

Diabetes mellitus, commonly referred to as diabetes, is a group of metabolic diseases in which there are high blood sugar levels over a prolonged period. Several types of diabetes exist, including type I diabetes, type II diabetes and gestational diabetes. Type 1 diabetes is characterized by loss of the insulin-producing β cells of the islets of Langerhans in the pancreas, leading to insulin deficiency. Type 2 diabetes is characterized by insulin resistance, which may be combined with relatively reduced insulin secretion. The defective responsiveness of body tissues to insulin is believed to involve the insulin receptor. Gestational diabetes, which resembles type 2 diabetes, occurs in about 2-10% of all pregnancies. The type of diabetes can also be classified as insulin-dependent diabetes mellitus, non-insulin dependent diabetes mellitus, malnutrition-related diabetes mellitus or unspecified diabetes mellitus.

The methods disclosed herein can be used to obtain a cell population enriched for insulin-producing β-cells. Accordingly, in some embodiments of the present disclosure there is provided a cell population for treatment of a metabolic disorder in an individual in need thereof. In some embodiments of the present disclosure, the metabolic disorder is selected from the group consisting of diabetes mellitus or a disorder of pancreatic internal secretion. In some embodiments of the present disclosure, the metabolic disorder is diabetes mellitus, such as insulin-dependent diabetes mellitus, non-insulin dependent diabetes mellitus, malnutrition-related diabetes mellitus or unspecified diabetes mellitus.

In one aspect of the present disclosure is provided a method of treatment of a metabolic disorder in an individual in need thereof, wherein the method comprises a step of providing a cell population enriched for insulin-producing β-cells obtainable by the methods described herein.

In some embodiments of the present disclosure, the present methods comprise a step of transplanting at least part of said enriched cell population into the individual suffering from a metabolic disorder.

In some embodiments of the present disclosure, the enriched cell population has increased expression of at least one of insulin, Insm-1, Isl-1, MafA, MafB or C-peptide compared to cell populations obtained from cells where Yap1 has not been inactivated, as described above. In some embodiments of the present disclosure, the enriched cell population has increased insulin area compared to cell populations obtained from cells where Yap1 has not been inactivated, as described above.

In some embodiments of the present disclosure, the cell population comprises at least 5% insulin-producing β-cells, such as at least 10% insulin-producing β-cells, such as at least 15% insulin-producing β-cells, such as at least 20% insulin-producing β-cells, such as at least 25% insulin-producing β-cells, such as at least 30% insulin-producing β-cells, such as at least 35% insulin-producing β-cells, such as at least 40% insulin-producing β-cells, such as at least 45% insulin-producing β-cells, such as at least 50% insulin-producing β-cells, such as at least 55% insulin-producing β-cells, such as at least 60% insulin-producing β-cells, such as at least 65% insulin-producing β-cells, such as at least 70% insulin-producing β-cells, such as at least 75% insulin-producing β-cells, or more.

### Yap1 inhibitor for use as a medicament

In one aspect, the present disclosure relates to the use of a Yap1 inhibitor for treatment of a metabolic disorder in an individual in need thereof.

The metabolic disorder can be any metabolic disorder as described above. Preferably, the disorder is related to a pancreatic disorder. Examples of metabolic disorders are: diabetes mellitus, including type 1 and type 2 diabetes.

In some embodiments of the present disclosure, the Yap1 inhibitor is verteporfin. Accordingly, there is disclosed the use of verteporfin for treatment of a metabolic disorder in an individual in need thereof.

In some embodiments of the present disclosure, the use of verteporfin for treatment of a metabolic disorder involves the methods described herein above, i.e. differentiation of cells into insulin-producing β-cells by incubating said cells in the presence of a Yap1 inhibitor.

### Identification of Yap1 inhibitor

In yet another aspect, the disclosure relates to a method for identifying a Yap1 inhibitor, said method comprising the steps of:
i) Providing a compound;
ii) Incubating said compound in the presence of Yap1;
iii) Measuring Yap1 activity and determining whether said compound is a Yap1 inhibitor.

### Examples

### Example 1 - introduction

Diabetes is a chronic metabolic disease affecting around 387 million people worldwide, counts to 1 in every 12 individuals, according to the world diabetes atlas. The disease symptoms include patients having high blood glucose levels, either because of inadequate production of insulin or improperly responding body cells to insulin or both. Treating diabetes could be done either by regeneration or transplantation of insulin producing islet cells. Human islet transplantation has been the promising therapeutic option for diabetes type 1 patients compared to insulin therapy, however it is limited by its scarcity on organ donations, challenges with isolation of islets from the pancreas and life-long use of immune suppressive drugs.

However, generation of pancreatic progenitors or insulin producing beta cells from human embryonic stem cells can be an alternative and promising source for transplantation that could extend this therapy to millions of new patients. These in vitro derived beta cells can also be used in *in vitro* drug screenings.

Although there are a number of reported protocols for the derivation of pancreatic endoderm from differentiating human pluripotent stem cells, generating functional insulin producing mature beta cells is still an unsolved question. Particularly the factors controlling the last step of differentiation, in which pancreatic progenitor cells are converted to functional insulin expressing cells, are still incompletely characterized.

We have discovered that when knocking out Yap1 in mice in the pancreatic progenitor cells that express Pdx1 gene, the progenitors differentiate towards endocrine fate, specifically to insulin producing beta cells. The knockout mice are hypo-glycaemic (less blood glucose levels), have high insulin area compared to controls, and express more insulin transcript. This unique phenotype leads to generation of more insulin producing functional and mature beta cells in-vivo.

Furthermore with this strong genetic evidence, we applied this strategy in human ES cells by pharmacologically inhibiting YAP1 activity (using YAP-TEAD inhibitor Verteporfin) in human ES cell derived pancreatic progenitors expressing PDX1. After 5 days of inhibitor treatment the pancreatic progenitors converted to insulin and MAFA expressing beta cells - the latter indicates that the cells reached a mature state which usually is associated with glucose-responsive insulin release.

### Example 2 - Conditional knockouts for Yap1 in mouse pancreas leads to hypoplasia and hypoglycaemia

The immunostainings and western blot data (not shown) for Yap1 indicate that Yap1 is highly expressed both at mRNA and protein levels in Pdx1 positive progenitor cells during early stage of mouse pancreas development, indicating the importance of this molecule during pancreas specification, maintenance and expansion of progenitors.

In order to address the importance of Yap1 in pancreas development, we utilized loss-of-function approach, to conditionally delete Yap1 in Pdx1 expressing cells using Pdx1 Cre mice that express Cre recombinase from an early embryonic stage (E9.5) in whole epithelium of pancreatic bud. Crossing the Yap1^{*fl*/*f*l} mice with Pdx1-Cre mice leads to pancreatic-specific Yap1 deficiency.

As expected the *Pdx1-Cre; Yap1*^{*fl*/*fl*} mice displayed a severe phenotype, which is pancreatic hypoplasia at Post-natal day 4 (Fig. 1). The size of the pancreas was reduced at least one third in Yap1 knockouts compared to the control littermates (Fig. 1B). The Knockout pups displayed reduced body weight (Fig. 1A and Fig. 1D), severe hypoglycaemia (Fig. 1C) and had not recovered after P6 day.

This is a unique and interesting phenotype where loss of a transcription factor Yap1 leads to hypoglycaemic nature.

### Example 3 - Yap1 knockout in mice

In order to understand further the Yap1 knockout phenotype in mice, we have performed immunostainings from the P4 pancreas and looked for expression of pancreatic genes. As shown in Figure 2 the Yap1 KO mice (Yap KO) displayed an increased area of insulin expression compared to controls despite their smaller size (Fig. 2A and fig. 2B). Quantification of the ratio of insulin-expressing cells to total DAPI area showed that the insulin area in the Yap KO mice was significantly higher than in the control mice. The insulin cell area was calculated from serial sections spanning the entire mouse pancreas tissue that were stained for insulin and DAPI (nuclei). The mean values of cross sectional expression areas from the acquired confocal images were measured using IMARIS software to get quantifications of insulin cell area. As expected the Yap1 mRNA levels were significantly down-regulated in the knockout pancreas compared to controls (Fig. 2E). The qRT PCR analysis revealed a significant up-regulation in insulin mRNA transcript in Yap KO (Fig. 2C) but the Glucagon mRNA levels were not significantly altered (Fig. 2D).

Collectively these data indicate that upon loss of Yap1 in Pdx1 expressing pancreatic progenitors, the cells tend to differentiate towards endocrine lineage and especially to Insulin producing beta cell fate.

### Example 4 - Loss of Yap1 in Sox9 expressing pancreatic progenitors leads to elevated insulin expression

We used a different Cre to delete Yap1 in pancreatic progenitors. Crossing Sox9 Cre ER (tamoxiferin induced Cre expression system) transgenic mice with Yap1 floxed mice results in specific activation of Cre in the Sox9 expressing pancreatic progenitors upon tamoxiferin treatment, thereby deleting Yap1 in those progenitors (figure 3B). This led to enhanced expression of Insulin as analysed by immune staining for Insulin as shown in figure 3A, confirmed by RT-qPCR (figure 3C). Insulin and glucagon expression was also found enhanced as shown by RT qPCR (figures 3D and 7E).
This example together with example 3 shows that the genetic knockout of Yap1 in multiple Cre expressing mouse lines Pdx1 or Sox9 leads to enhanced beta cell differentiation.

### Reference Example 5 - inhibition of YAP1 in hES cells

The genetic data from mouse revealed an unexpected and interesting role of Yap1 during pancreas development indicating the importance of Yap1 during differentiation of pancreatic progenitors towards insulin expressing Beta cells. The interesting findings in mice led us to propose and check the relevance of YAP1 in human embryonic stem cell derived pancreatic progenitors. Treating the PDX1 expressing hES derived pancreatic progenitors with a chemical Yap-TEAD inhibitor, Verteporfin, can mimic YAP1 loss of function in cultured cells. To this end we utilized a hES PDX1eGFP clone in which one allele of PDX1 was replaced by eGFP and the second allele was functional. These cells are PDX1 heterozygotic and express eGFP when PDX1 expression commences.

We cultured the hES PDX1 GFP cells using a modified protocol from Rezania et al 2010 (see below for the exact protocol, outlined in Fig. 5A) that efficiently generates pancreatic progenitors mostly expressing PDX1, NKX6.1. Briefly, Hues4 hES PDX1 GFP cells were cultured on feeder-free DEF system (""DEF-CS™", Cellectis stem cells, Cellartis AB") until full confluency. When full confluency was reached the cells were subjected to differentiation as follows for 5 different stages to obtain insulin producing mature Beta cells.

### Stage 1: Definitive Endoderm:

Cells were cultured in RPMI medium with 3 µM CHIR99021 (a Wnt activator) for 1 day. During the second day the cells were treated with 100 ng/ml Activin A. For days 3 and 4 the cells were treated with 100 ng/ml Activin A along with 2% (v/v) B27.

### Stage 2: Primitive Gut tube:

The cultures were exposed to F12 media containing 2% B27, 2 gm/L Sodium bicarbonate along with 0.25 mM Vitamin C and 50 ng/ml FGF7 for two days.

### Stage 3: Posterior Foregut:

The cultures were continued for four days in DMEM high glucose media along with 2% B27, 2 g/L Sodium bicarbonate, 0.25 mM Vitamin C, 2 ng/ml FGF7, 0.25 µM Sant1, 100 ng/ml Noggin and 2 µM Retinoic acid.

### Stage 4: Primitive Endoderm:

Stage 4 cells were cultured for 5 days to obtain PDX1 and NKX6.1 expressing pancreatic progenitor cells by culturing in 2% B27, 2 gm/L Sodium bicarbonate, 0.25 mM Vitamin C, 100 ng/ml Noggin and 500 nM TPB (PKC activator).

### Stage 5: mature Beta cells:

To obtain insulin expressing, MAFA positive mature Beta cells, the cultures were treated with the YAP-TEAD inhibitor Verteporfin at a concentration of 1 µg/ml in Stage 4 medium and cultured for another 4-9 days.

After culturing the cells for the first 4 stages above (around 15 days), the differentiated hES cells were enriched for PDX1 expression as they expressed GFP. These enriched pancreatic progenitor cultures were treated with the YAP1 -TEAD chemical inhibitor Verteporfin for an additional 4-5 days and checked for gene expression (stage 5).

As expected from mouse studies, inhibition of YAP1 activity in the PDX1 enriched cells, using verteporfin after 4 days led to efficient and enhanced endocrine differentiation especially towards insulin producing beta cells at the expense of acinar lineage.

The qRT PCR analysis of 4 day verteporfin-treated cultures (Fig. 5B-5G) showed up-regulation of endocrine genes such as Insulin, Glucagon, ISL-1, MAFA and MAFB, while exocrine genes like CPA1 and PTF1a were down-regulated compared to DMSO treated control cultures. These results indicate that when YAP1 is inhibited in pancreatic progenitors, these progenitors show enhanced differentiation towards endocrine cell fate.

Furthermore, the qRT PCR analysis of 5 day verteporfin-treated cultures (Fig. 6) showed that these cells undergo better endocrine differentiation as they consistently displayed up-regulation of beta cell maturation markers like MAFA, ISL1 and INSM1 compared to day 4 cultures. The verteporfin treatment directed the pancreatic progenitors to endocrine lineage differentiation at the expense of acinar lineage. The insulin mRNA levels were 5 fold up-regulated compared to DMSO control cultures.
Cells treated with DMSO or verteporfin for 5 days and immunostained with C-peptide, Glucagon and GFP antibodies (Figure 7) showed that insulin (c-peptide) and Glucagon protein expression was enhanced in verteporfin-treated cells. These Insulin expressing cells were positive for MAFA, indicating that these cells are mature and functional.

Altogether, these results support our findings in mice, where loss/inhibition of Yap1 in Pdx1 pancreatic progenitor cells led to enhanced endocrine differentiation, specifically insulin expressing and mature beta cells.

### Example 6 - inhibition of YAP1 in mES cells

Wild type C57bl6 mouse pancreatic explants at E11.5 embryonic stages were cultured ex-vivo for initial 24 hours in normal culture media supplemented with 1 µg/ml Verteporfin for 72 hours and again in normal culture media without Verteporfin for another 48 hours. The explants were analyzed by RT-qPCR for endocrine gene expression after 6 days of culture. Inhibition of Yap1 using Verteporfin led to significant up-regulation of endocrine progenitors Neurogenin (Ngn3) (figure 4A), NeuroD (figure 4B), Insm1 (figure 4C) and Arx expression (figure 4D). Expression of these genes is characteristic of islet cell fate. This ultimately led to enhanced endocrine differentiation as shown by increased expression of Insulin (figure 4E) and Glucagon (figure 4F).

This example together with example 4 shows that chemical inhibition of Yap1 leads to enhanced beta cell differentiation in mouse pancreas.

### Reference Example 7 - hES cell derived pancreatic progenitors treated with VP for 5 days show enhanced endocrine differentiation & Insulin expression.

Stable Pdx1 Gfp human ES cells were differentiated to pancreatic progenitor stage and then treated with either DMSO (control) or Verteporfin (VP, 1 µg/ml) for 5 additional days. Cultures were immuno-stained with c-peptide and Neurogenin3 antibodies and imaged using confocal microscopy (figure 8). Verteporfin treated cultures exhibited enhanced endocrine and beta cell differentiation and expressed higher levels of Ngn3 and c-peptide compared to DMSO treated cells.

### Reference Example 8 - Transient loss of Yap1 in Pdx1 GFP hES cells leads to up-regulation of endocrine progenitor gene expression.

Stable Pdx1 Gfp human ES cells were differentiated to pancreatic progenitor stage and then transiently transfected with control or Yap1 siRNA. Cultures were analyzed using RT-qPCR after 3 days of siRNA transfection. As shown in figure 9, cells transfected with Yap1 siRNA showed 50% reduction in Yap1 mRNA levels (figure 9A) and this results in increased expression of the endocrine progenitors Ngn3 (figure 9B) and Insm1 (figure 9C).

This example confirms that inactivation of Yap1 leads to enhanced beta cell differentiation.

### References

Aoi, T. et al. (2008) Nihon Rinsho. 66(5):850-6
Chung et al. (2008) Cell Stem Cell. 2(2):113-7.
D'Amour, K. A. et al. (2006) Nat Biotechnol. (11): 1392-401.
Heins et.al. (2004) Stem Cells. 22(3):367-76.
Jiang, J. et al. (2007), Stem Cells 25
Kroon, E. et al. (2008) Nat Biotechnol. (4):443-52.
Pagliuca et al. (2014) Cell. 159(2):428-39
Rezania etal, (2010) Eur J Pharmacol. 2010 Feb 10;627(1-3):265-8
Rezania et al. (2012) Diabetes. 2012 Aug;61(8):2016-29
Rezania et al. (2014) Nat Biotechnol. (32):1121-33.
Shapiro et al, (2000) N Engl J Med 343:230-238
Shapiro et al, (2001a) Best Pract Res Clin Endocrinol Metab 15:241- 264
Shapiro et al, (2001b) British Medical Journal 322:861
Stadtfeld and Hochedlinger (2010) Genes Dev. 24(20):2239-63
Takahashi and Yamanaka (2006) Cell. 2006 Aug 25;126(4):663-76
Takahashi et al. (2007) Cell 131 (5):861
Takashima et al. (2014) Cell. 158(6): 1254-1269
Tesar et al. (2007) Nature 448(7150):196-9
Thomson, A. et al. (1998) Science. 6;282(5391):1145-7.
Wernig, M. et al. (2007) Nature. 448(7151):318-24
Yu et al., (2007) Science 318:5858
Yu J, et al. (2009) Science vol 324

## Claims

1. An in vitro method for differentiation of cells into insulin-producing β-cells, said method comprising the steps of:
i) inactivating Yap1 in a pancreatic progenitor cell population comprising at least one cell capable of differentiation;
ii) differentiating the cells of step i) and committing them to endocrine fate, preferably to insulin producing β-cell fate;
thereby obtaining a cell population enriched for insulin-producing β-cells, compared to cells where Yap1 was not inactivated.

2. The method according to claim 1, wherein the insulin-producing β-cells are insulin-responsive.

3. The method according to any one of the preceding claims, wherein the pancreatic progenitor cell population is inactivated with a Yap1 inhibitor and the cell population enriched for insulin-producing β-cells is capable of producing at least 1.5-fold more insulin than cells obtained when the cell population of step i) is incubated in the absence of Yap1 inhibitor, optionally at least 1.6-fold more insulin, such as at least 1.7-fold more insulin, such as at least 1.8-fold more insulin, such as at least 1.8-fold more insulin, such as at least 1.9-fold more insulin, such as at least 2.0-fold more insulin, such as at least 2.5-fold more insulin, such as at least 3.0-fold more insulin, such as at least 3.5-fold more insulin, such as at least 4.0-fold more insulin, such as at least 4.5-fold more insulin, such as at least 5.0-fold more insulin, such as at least 5.5-fold more insulin, such as at least 6.0-fold more insulin, such as at least 6.5-fold more insulin, such as at least 7.0-fold more insulin, such as at least 7.5-fold more insulin, such as at least 8.0-fold more insulin, such as at least 8.5-fold more insulin, such as at least 9.0-fold more insulin, such as at least 9.5-fold more insulin, such as at least 10-fold more insulin, such as at least 12.5-fold more insulin, such as at least 15-fold more insulin, such as at least 17.5-fold more insulin, such as at least 20-fold more insulin, such as at least 25-fold more insulin compared to a cell population incubated in the absence of Yap1 inhibitor.

4. The method according to any one of the preceding claims, wherein the pancreatic progenitor cell population is a cell population enriched for bona fide pancreatic progenitor cells obtained by a method comprising exposing a cell population comprising at least one bona fide pancreatic progenitor cell, wherein the bona fide pancreatic progenitor cell expresses PDX1 and NKX6-1 to:
a) a first ligand which binds to a first marker specific for PDX1⁻ cells and selecting the cells that do not bind to said first ligand from said cell population, thereby enriching the cell population for PDX1⁺ cells;
and/or
b) a second ligand which binds to a second marker specific for PDX1⁺ cells and selecting the cells that bind to said second ligand from the cells that do not bind to said second ligand, thereby enriching the cell population for PDX1⁺ cells;
and/or
c) a third ligand which binds to a third marker specific for PDX1⁺ NKX6-1⁺ cells and selecting the cells that bind to said third ligand from the cells that do not bind to said third ligand, thereby enriching the cell population for PDX1⁺ NKX6-1⁺ cells;
thereby obtaining a cell population enriched for bona fide pancreatic progenitor cells.

5. The method according to claim 4, wherein the third ligand is an antibody or fragment thereof directed against GP2.

6. The method according to any one of claims 4 to 5, wherein the first ligand is an antibody or fragment thereof directed against CD49d and the third ligand is an antibody directed against GP2.

7. The method according to any one of claims 4 to 6, wherein the first ligand is an antibody or fragment thereof directed against CD49d, the second ligand is an antibody or fragment thereof directed against FOLR1 and the third ligand is an antibody or fragment thereof directed against GP2.

8. The method according to any one of claims 4 to 7, wherein the bona fide pancreatic progenitor cells are derived from cells capable of differentiation such as human pluripotent stem cells, such as human iPS cells (hIPSCs), human ES cells (hESCs) and naive human stem cells (NhSCs).

9. An in vitro method for increasing endocrine differentiation, said method comprising the steps of:
i) inactivating Yap1 in a pancreatic progenitor cell population comprising at least one cell capable of differentiation;
ii) differentiating the cells of step i) toward endocrine fate;
thereby obtaining a cell population enriched for endocrine cells, compared to cells where Yap1 was not inactivated.

10. A Yap1 inhibitor for use in the treatment of a metabolic disorder in an individual in need thereof, wherein the metabolic disorder preferably is diabetes mellitus, such as insulin-dependent diabetes mellitus, such as non-insulin-dependent diabetes mellitus, such as malnutrition-related diabetes mellitus.

11. The Yap1 inhibitor for the use according to claim 10, wherein the Yap1 inhibitor is verteporfin.

## Patentansprüche

1. In-vitro-Verfahren zur Differenzierung von Zellen zu insulinproduzierenden β-Zellen, wobei das Verfahren die folgenden Schritte umfasst:
i) Inaktivieren von Yap1 in einer Pankreas-Vorläuferzellpopulation, die mindestens eine differenzierungsfähige Zelle umfasst;
ii) Differenzieren der Zellen von Schritt i) und Festlegen dieser auf ein endokrines Schicksal, vorzugsweise auf ein insulinproduzierendes β-Zell-Schicksal;
dadurch Erhalten einer Zellpopulation, die im Vergleich zu Zellen, in denen Yap1 nicht inaktiviert war, mit insulinproduzierenden β-Zellen angereichert ist.

2. Verfahren nach Anspruch 1, wobei die insulinproduzierenden β-Zellen auf Insulin ansprechen.

3. Verfahren nach einem der vorhergehenden Ansprüche, wobei die Pankreas-Vorläuferzellpopulation mit einem Yap1-Inhibitor inaktiviert ist und die mit insulinproduzierenden β-Zellen angereicherte Zellpopulation fähig ist, mindestens 1,5-fach mehr Insulin zu produzieren als Zellen, die erhalten werden, wenn die Zellpopulation von Schritt i) in Abwesenheit eines Yap1-Inhibitors inkubiert wird, optional mindestens 1,6-fach mehr Insulin, wie etwa mindestens 1,7-fach mehr Insulin, wie etwa mindestens 1,8-fach mehr Insulin, wie etwa mindestens 1,8-fach mehr Insulin, wie etwa mindestens 1,9-fach mehr Insulin, wie etwa mindestens 2,0-fach mehr Insulin, wie etwa mindestens 2,5-fach mehr Insulin, wie etwa mindestens 3,0-fach mehr Insulin, wie etwa mindestens 3,5-fach mehr Insulin, wie etwa mindestens 4,0-fach mehr Insulin, wie etwa mindestens 4,5-fach mehr Insulin, wie etwa mindestens 5,0-fach mehr Insulin, wie etwa mindestens 5,5-fach mehr Insulin, wie etwa mindestens 6,0-fach mehr Insulin, wie etwa mindestens 6,5-fach mehr Insulin, wie etwa mindestens 7,0-fach mehr Insulin, wie etwa mindestens 7,5-fach mehr Insulin, wie etwa mindestens 8,0-fach mehr Insulin, wie etwa mindestens 8,5-fach mehr Insulin, wie etwa mindestens 9,0-fach mehr Insulin, wie etwa mindestens 9,5-fach mehr Insulin, wie etwa mindestens 10-fach mehr Insulin, wie etwa mindestens 12,5-fach mehr Insulin, wie etwa mindestens 15-fach mehr Insulin, wie etwa mindestens 17,5-fach mehr Insulin, wie etwa mindestens 20-fach mehr Insulin, wie etwa mindestens 25-fach mehr Insulin im Vergleich zu einer in Abwesenheit eines Yap1-Inhibitors inkubierten Zellpopulation.

4. Verfahren nach einem der vorhergehenden Ansprüche, wobei die Pankreas-Vorläuferzellpopulation eine Zellpopulation ist, die mit bona fiden Pankreas-Vorläuferzellen angereichert ist, die durch ein Verfahren erhalten wurden, das das Aussetzen einer Zellpopulation umfassend mindestens einer bona fiden Pankreas-Vorläuferzelle dem Nachfolgenden umfasst, wobei die bona fide Pankreas-Vorläuferzelle PDX1 und NKX6-1 exprimiert:
a) einem ersten Liganden, der an einen ersten Marker bindet, der für PDX1⁻-Zellen spezifisch ist, und Auswählen der Zellen, die nicht an den ersten Liganden binden, aus der Zellpopulation, wodurch die Zellpopulation mit PDX1⁺-Zellen angereichert wird;
und/oder
b) einem zweiten Liganden, der an einen zweiten Marker bindet, der für PDX1⁺-Zellen spezifisch ist, und Auswählen der Zellen, die an den zweiten Liganden binden, aus den Zellen, die nicht an den zweiten Liganden binden, wodurch die Zellpopulation mit PDX1⁺-Zellen angereichert wird;
und/oder
c) einem dritten Liganden, der an einen dritten Marker bindet, der für PDX1⁺-NKX6-1⁺-Zellen spezifisch ist, und Auswählen der Zellen, die an den dritten Liganden binden, aus den Zellen, die nicht an den dritten Liganden binden, wodurch die Zellpopulation mit PDX1⁺-NKX6-1⁺-Zellen angereichert wird;
dadurch Erhalten einer Zellpopulation, die mit bona fiden Pankreas-Vorläuferzellen angereichert ist.

5. Verfahren nach Anspruch 4, wobei der dritte Ligand ein gegen GP2 gerichteter Antikörper oder ein Fragment davon ist.

6. Verfahren nach einem der Ansprüche 4 bis 5, wobei der erste Ligand ein gegen CD49d gerichteter Antikörper oder ein Fragment davon ist und der dritte Ligand ein gegen GP2 gerichteter Antikörper ist.

7. Verfahren nach einem der Ansprüche 4 bis 6, wobei der erste Ligand ein gegen CD49d gerichteter Antikörper oder ein Fragment davon ist, der zweite Ligand ein gegen FOLR1 gerichteter Antikörper oder ein Fragment davon ist und der dritte Ligand ein gegen GP2 gerichteter Antikörper oder ein Fragment davon ist.

8. Verfahren nach einem der Ansprüche 4 bis 7, wobei die bona fiden Pankreas-Vorläuferzellen von differenzierungsfähigen Zellen wie etwa humanen pluripotenten Stammzellen, wie etwa humanen iPS-Zellen (hIPSC), humanen ES-Zellen (hESC) und naiven humanen Stammzellen (NhSC) abgeleitet sind.

9. In-vitro-Verfahren zum Erhöhen der endokrinen Differenzierung, wobei das Verfahren die folgenden Schritte umfasst:
i) Inaktivieren von Yap1 in einer Pankreas-Vorläuferzellpopulation, die mindestens eine differenzierungsfähige Zelle umfasst;
ii) Differenzieren der Zellen von Schritt i) in Richtung endokrinem Schicksal;
dadurch Erhalten einer Zellpopulation, die im Vergleich zu Zellen, in denen Yap1 nicht inaktiviert war, mit endokrinen Zellen angereichert ist.

10. Yap1-Inhibitor zur Verwendung bei der Behandlung einer Stoffwechselstörung bei einer Person, die dies benötigt, wobei die Stoffwechselstörung vorzugsweise Diabetes mellitus wie etwa insulinabhängiger Diabetes mellitus, wie etwa nichtinsulinabhängiger Diabetes mellitus, wie etwa mangelernährungsbedingter Diabetes mellitus ist.

11. Yap1-Inhibitor zur Verwendung nach Anspruch 10, wobei der Yap1-Inhibitor Verteporfin ist.

## Revendications

1. Procédé in vitro de différenciation de cellules en cellules β productrices d'insuline, ledit procédé comprenant les étapes :
i) d'inactivation de Yap1 dans une population de cellules progénitrices pancréatiques comprenant au moins une cellule capable de différenciation ;
ii) de différenciation des cellules de l'étape i) et de soumission de celles-ci à un destin endocrinien, de préférence à un destin de cellules β productrices d'insuline ;
obtenant ainsi une population de cellules enrichie en cellules β productrices d'insuline, par rapport à des cellules où Yap1 n'était pas inactivé.

2. Procédé selon la revendication 1, dans lequel les cellules β productrices d'insuline sont sensibles à l'insuline.

3. Procédé selon l'une quelconque des revendications précédentes, dans lequel la population de cellules progénitrices pancréatiques est inactivée avec un inhibiteur de Yap1 et la population de cellules enrichie en cellules β productrices d'insuline est capable de produire au moins 1,5 fois plus d'insuline que les cellules obtenues lorsque la population de cellules de l'étape i) est incubée en l'absence d'inhibiteur de Yap1, éventuellement au moins 1,6 fois plus d'insuline, comme au moins 1,7 fois plus d'insuline, comme au moins 1,8 fois plus d'insuline, comme au moins 1,8 fois plus d'insuline, comme au moins 1,9 fois plus d'insuline, comme au moins 2,0 fois plus d'insuline, comme au moins 2,5 fois plus d'insuline, comme au moins 3,0 fois plus d'insuline, comme au moins 3,5 fois plus d'insuline, comme au moins 4,0 fois plus d'insuline, comme au moins 4,5 fois plus d'insuline, comme au moins 5,0 fois plus d'insuline, comme au moins 5,5 fois plus d'insuline, comme au moins 6,0 fois plus d'insuline, comme au moins 6,5 fois plus d'insuline, comme au moins 7,0 fois plus d'insuline, comme au moins 7,5 fois plus d'insuline, comme au moins 8,0 fois plus d'insuline, comme au moins 8,5 fois plus d'insuline, comme au moins 9,0 fois plus d'insuline, comme au moins 9,5 fois plus d'insuline, comme au moins 10 fois plus d'insuline, comme au moins 12,5 fois plus d'insuline, comme au moins 15 fois plus d'insuline, comme au moins 17,5 fois plus d'insuline, comme au moins 20 fois plus d'insuline, comme au moins 25 fois plus d'insuline par rapport à une population de cellules incubée en l'absence d'inhibiteur de Yap1.

4. Procédé selon l'une quelconque des revendications précédentes, dans lequel la population de cellules progénitrices pancréatiques est une population de cellules enrichie en cellules progénitrices pancréatiques authentiques obtenues par un procédé comprenant
l'exposition d'une population de cellules comprenant au moins une cellule progénitrice pancréatique authentique, dans lequel la cellule progénitrice pancréatique authentique exprime PDX1 et NKX6-1 à :
a) un premier ligand qui se lie à un premier marqueur spécifique pour les cellules PDX1⁻, et la sélection des cellules qui ne se lient pas audit premier ligand à partir de ladite population de cellules, enrichissant ainsi la population de cellules pour les cellules PDX1⁺ ;
et/ou
b) un deuxième ligand qui se lie à un deuxième marqueur spécifique pour les cellules PDX1⁺, et la sélection des cellules qui se lient audit deuxième ligand par rapport aux cellules qui ne se lient pas audit deuxième ligand, enrichissant ainsi la population de cellules pour les cellules PDX1⁺ ;
et/ou
c) un troisième ligand qui se lie à un troisième marqueur spécifique pour les cellules PDX1⁺ NKX6-1⁺, et la sélection des cellules qui se lient audit troisième ligand par rapport aux cellules qui ne se lient pas audit troisième ligand, enrichissant ainsi la population de cellules pour les cellules PDX1⁺ NKX6-1⁺ ;
obtenant ainsi une population de cellules enrichie en cellules progénitrices pancréatiques authentiques.

5. Procédé selon la revendication 4, dans lequel le troisième ligand est un anticorps ou un fragment de celui-ci dirigé contre GP2.

6. Procédé selon l'une quelconque des revendications 4 à 5, dans lequel le premier ligand est un anticorps ou un fragment de celui-ci dirigé contre CD49d et le troisième ligand est un anticorps dirigé contre GP2.

7. Procédé selon l'une quelconque des revendications 4 à 6, dans lequel le premier ligand est un anticorps ou un fragment de celui-ci dirigé contre CD49d, le deuxième ligand est un anticorps ou un fragment de celui-ci dirigé contre FOLR1 et le troisième ligand est un anticorps ou un fragment de celui-ci dirigé contre GP2.

8. Procédé selon l'une quelconque des revendications 4 à 7, dans lequel les cellules progénitrices pancréatiques authentiques sont dérivées de cellules capables de différenciation telles que des cellules souches pluripotentes humaines, telles que des cellules iPS humaines (hIPSC), des cellules ES humaines (hESC) et des cellules souches humaines naïves (NhSC).

9. Procédé in vitro pour augmenter la différenciation endocrinienne, ledit procédé comprenant les étapes :
i) d'inactivation de Yap1 dans une population de cellules progénitrices pancréatiques comprenant au moins une cellule capable de différenciation ;
ii) de différenciation des cellules de l'étape i) vers le destin endocrinien ;
permettant ainsi d'obtenir une population de cellules enrichie en cellules endocriniennes, par rapport à des cellules où Yap1 n'était pas inactivé.

10. Inhibiteur de Yap1 destiné à être utilisé dans le traitement d'un trouble métabolique chez un individu qui en a besoin, dans lequel le trouble métabolique est de préférence les diabètes sucrés, tels que les diabètes sucrés insulinodépendants, tels que les diabètes sucrés non insulinodépendants, tels que les diabètes sucrés liés à la malnutrition.

11. Inhibiteur de Yap1 pour l'utilisation selon la revendication 10, dans lequel l'inhibiteur de Yap1 est la vertéporfine.
